# EUROPEAN PATENT SPECIFICATION

(11) **EP 3 438 280 B1**
(45) Date of publication and mention of the grant of the patent: **25.11.2020**
(21) Application number: 17184008.5
(22) Date of filing: 31.07.2017
(51) Int. Cl.: C12Q 1/68

(54) **HAEMOPLASMA DETECTION METHOD**
HÄMOPLASMADETEKTIONSVERFAHREN
PROCÉDÉ DE DÉTECTION D'HÉMOPLASME

(43) Date of publication of application: 06.02.2019
(73) Proprietor: Rosengarten, Renate, 1010 Vienna (AT)
(72) Inventor: ZIMMERMAN, Carl-Ulrich Richard, 1110 Vienna (AT); ROSENGARTEN, Renate, 1010 Vienna (AT)
(74) Representative: Redl, Gerda

(56) References cited:
- JP-A- 2012 060 925
- US-A1- 2006 252 080
- B. WILLI ET AL: "Development and Application of a Universal Hemoplasma Screening Assay Based on the SYBR Green PCR Principle", JOURNAL OF CLINICAL MICROBIOLOGY, vol. 47, no. 12, 14 October 2009 (2009-10-14), pages 4049-4054, XP055407986, US ISSN: 0095-1137, DOI: 10.1128/JCM.01478-09
- TASKER ET AL.: JOURNAL OF MEDICAL MICROBIOLOGY, vol. 59, 2010, pages 1285-1292, XP002773939,

## Description

The invention relates to a molecular technique and assay to detect bacteria of the haemoplasma group of mycoplasmas with a broad coverage.

### BACKGROUND

Haemotropic mycoplasmas, also known as haemoplasmas or haemoplasma bacteria, are small, pleomorphic, cell wall-free bacteria that have been detected in the blood of various mammalian species. Though many haemoplasmas are of non-human animal origin, there is a high zoonotic potential, possibly transmitting disease to humans. Haemoplasmas are divided based on phylogeny into two groups: a haemominutum group and a haemofelis group.

Blood products or blood components including e.g. red cells, platelets, fresh frozen plasma and cryoprecipitate, but also plasma derivatives manufactured from pooled plasma donations, such as albumin, coagulation factors and immunoglobulins, originate from donors and potentially contain pathogens. Such products need to be safely used in clinical practice.

Blood for transfusion is a potential source of infection by a variety of known and unknown transmissible agents. While the risk of viral infection via allogeneic blood has been substantially reduced through early detection of blood-borne viral contamination in the source plasma and final products, bacterial contamination of blood products has emerged as source of transfusion-transmitted disease.

Mycoplasma contamination is also a major challenge in cell culture techniques, used in the production of recombinant proteins. Mycoplasma contamination is invisible with no discernible change in turbidity or pH, and can be extremely virulent.

Willi et al. (Journal of Clinical Microbiology 2009, 47: 4049-4054) describe the development of a universal haemoplasma PCR screening assay as a diagnostic tool to analyse human blood sample. The PCR assay is based on the 16S rRNA gene amplification and covers haemotropic mycoplasmas of feline, bovine, porcine, camelid and murine origin, but also cross-reacts with non-haemotropic mycoplasma species, such as *Mycoplasma penetrans* and *Mycoplasma pneumoniae.*

Tasker et al. (Journal of Medical Microbiology 2010, 59:1285-1292) describe the determination of human haemotropic mycoplasma infection using two generic haemoplasma real-time PCR assays (qPCR) on blood samples and blood smears. One of the two generic haemoplasma qPCRs covers the haemofelis group, and the other one covers the haemominutum group. No cross-reactivity was observed with any non-target mycoplasma species.

### SUMMARY OF THE INVENTION

It is the objective of the present invention to provide an improved generic qPCR assay that broadly covers haemotropic mycoplasmas including the major species of both, the haemofelis group and the haemominutum group.

The objective is solved by the subject of the present invention as described herein.

According to the invention, there is provided a method for detecting at least one haemoplasma species within the group of haemoplasmas of the genus *Mycoplasma,* in particular any haemoplasma species within the entire group of haemoplasmas of the genus *Mycoplasma,* in a biological sample, said method comprising the steps of:
i) treating said sample to release haemoplasma nucleic acid in the sample,
ii) amplifying a segment of a 16S rRNA gene by real-time PCR (qPCR) using an oligonucleotide primer pair, said oligonucleotide primer pair consisting of a forward primer comprising at least 10 consecutive bases of SEQ ID 1 and a reverse primer comprising at least 10 consecutive bases of SEQ ID 2, thereby producing an amplification product, and
iii) detecting the amplification product, herein also referred to as "amplicon", by means producing a detection signal correlating with the amount of the amplification product which is indicative of at least one or any haemoplasma species within the genus *Mycoplasma* (such genus is herein understood to encompass the *Mycoplasma* species and Candidatus *Mycoplasma* species, in particular those covered by the method described herein).

Specifically, the forward primer comprises or consists of SEQ ID 1, and the reverse primer comprises or consists of SEQ ID 2.
SEQ ID 1: AGCAAATGGGATTAGATACCCCA
SEQ ID 2: TTCCTTTGAGTTTCATATTTGCATA

Specifically, the amplification product is indicative of a group of different haemoplasma species at least comprising haemoplasmas of the haemofelis group and the haemominutum group. In particular, the method described herein covers at least the following haemoplasma species: *Mycoplasma haemofelis, Mycoplasma haemocanis, Mycoplasma haemomuris, Candidatus* Mycoplasma haemohominis, *Candidatus* Mycoplasma turicensis, *Mycoplasma coccoides, Candidatus* Mycoplasma haemomacaque, *Candidatus* Mycoplasma haemobos, *Candidatus* Mycoplasma haemovis, *Mycoplasma ovis, Mycoplasma parvum, Candidatus* Mycoplasma haemominutum, *Candidatus* Mycoplasma haematoparvum, *Candidatus* Mycoplasma kahanei, Candidatus Mycoplasma haemozalophi, Candidatus Mycoplasma haemolamae, *Mycoplasma suis, Candidatus* Mycoplasma haemodidelphis, *Mycoplasma wenyonii, Candidatus* Mycoplasma erythrocervae, and *Candidatus* Mycoplasma haemocervae.

Specifically, the method described herein, also referred to as "test", is not cross-reactive with *Mycoplasma penetrans* or *Mycoplasma pneumoniae,* or any other non-haemotropic mycoplasma species. More specifically, the test is not cross-reactive with any of the following mycoplasma species: *M. arginini,M. bovigenitalium M. bovirhinis, M. bovoculi, M. californicum, M. canis, M. canadense, M. cynos, M. dispar, M. edwardii, M. fastidiosum, M. felis, M. fermentans, M. gallisepticum, M*. *gateae, M. hominis, M. hyopneumoniae, M. hyorhinis, M. maculosum, M. molare, M. mucosicanis, M. mycoides* spp. *mycoides* SC, *M. orale, M. pneumoniae, M. salivarium, M. synoviae, Acholeplasma laidlawii,* and *Spiroplasma citri.* Genomic DNA of type or field strains of the listed mycoplasma species with a concentration of 10⁶ copies can be analyzed with the generic haemoplasma qPCR test. Non-crossreactivity is proven, if no amplifications (or less than 1 genome equivalent [see Table 3]) are determined at the given concentration.

Specifically, the amplification product is detected by a quantitative detection method.

Specifically, the lower detection limit is below 6 copies/ PCR. For example, a set of 10-fold serial dilutions of purified and linearized (e.g., Mhmu) control plasmid DNA (copy numbers ranging from 1 to 10⁶ copies per PCR reaction) was amplified yielding positive results for all concentrations of the control DNA. To determine the lower detection limit of the PCR, all control plasmids were diluted in 10-fold serial dilutions and compared to a standard curve of the control plasmid (Mhmu). The lowest copy numbers to be detected ranged from 0.18 to 5.83, thus the lowest detection limit of the PCR was below 1 copy per reaction.

Specifically, the sensitivity for diagnostic sample material is at least 10 copies per 1 ml of whole blood. For example, three product matrices (rabbit whole blood, human whole blood and beagle whole blood) were spiked with different concentrations of the (e.g., Mhmu) control plasmid. Spike levels ranged from 10⁶ to 10 copies per ml product matrix. DNA was extracted from 1 ml of each spiked or unspiked sample and eluted with 100 µl DNA extraction buffer. Per PCR reaction, 10 µl DNA eluate was analyzed. In all three product matrices, the 10 copies per ml spike level was detected. Thus, the detection-sensitivity of the assay was as low as 1 - 10 DNA copies per PCR reaction in a product matrix spiked with 10 DNA copies per ml sample. No cross reactions were observed with the unspiked blood samples.

Specifically, the quantitative detection method employs
i) a double-stranded DNA-binding dye (dsDNA dye) as reporter, preferably wherein the dsDNA dye is fluorescent and the amplification product is detected by measuring fluorescence; or
ii) at least one probe specifically annealing with the amplification product and comprising a detectable label, preferably wherein said at least one probe is an oligonucleotide consisting of SEQ ID 3.
   SEQ ID 3: 5'-CATCGTTTACGGTGTGGACTACTG-3'

Specifically, the probe is a labeled probe, e.g. fluorogenic probe.

A detectably labeled oligonucleotide probe is specifically used which is sufficiently complementary to and capable of hybridizing with the amplified nucleic acid (i.e. the amplicon), if present, as an indication of the presence or absence of any haemoplasma in the sample. A detectably labeled probe includes e.g. as a fluorescent label 6-carboxyfluorescein (FAM), tetramethyl rhodamine (TAMRA), 2', 4', 5', T-tetrachloro-4-7-dichlorofluorescein (TET), Cy5, 6-carboxy-X-rhodamine (ROX), CAL Fluor® Dyes (dye-CPGs (controlled pore glass) or dye-phosphoramidites) such as CAL Fluor® GOLD (LGC Biosearch Technologies); Molecular Beacons (which are oligonucleotides labeled with a fluorescent compound and a quencher (Tyagi S, Kramer FR: Molecular beacons: probes that fluoresce upon hybridization. Nat Biotechnol 1996, 14: 303-8); Scorpions Fluorescent Signaling Molecules (which are bifunctional molecules combining primer and probe in a single oligonucleotide, Carters et al., Methods Mol Biol. 2008; 429: 99-115).

Specifically, said dsDNA dye is any of SYBR™ green dye (N',N'-dimethyl-N-[4-[(E)-(3-methyl-1,3-benzothiazol-2-ylidene)methyl]-1-phenylquinolin-1-ium-2-yl]-N-propylpropane-1,3-diamine), or other asymmetrical cyanine dyes used as a nucleic acid stain in molecular biology, such as SYBR GOLD™ (ThermoFisher Scientific), and EVAGREEN™ (Biotium).

Specifically, said quantitative detection method is using real-time SYBR™ green PCR, or a fluorogenic 5' nuclease assay, such as the TaqMan™ technique. Specifically, said detection step may include fusion temperature analysis or melting curve analysis, e.g., where the melting point temperature is usually determined experimentally by subjecting the sample to a constitutive increase in temperature and continuously measuring the dissociation of the hybridization complex into single strands. The dissociation is detected by means of flourescence.

Specifically, said treating step i) in the method described herein, comprises lysing haemoplasma cells, nucleic acid extraction and purification, thereby obtaining purified haemoplasma nucleic acid in a buffer.

Specifically, the 16S rRNA gene is understood as the gene encoding 16S ribosomal RNA, which is the component of the 30S small subunit of a prokaryotic ribosome that binds to the Shine-Dalgarno sequence. The genes coding for it are referred to as 16S rRNA gene, also referred to as 16S DNA, and are used in reconstructing phylogenies, due to the slow rates of evolution of this region of the gene.

According to a specific aspect, said segment of the 16S rRNA gene is part of a cDNA obtained by reverse transcription of RNA into cDNA using a reverse transcriptase.

Specifically, said segment of the 16S rRNA gene comprises the nucleotide sequence identified as SEQ ID 4. Specifically, said amplification product comprises or consists of molecules characterized by the consensus sequence identified by SEQ ID 4.
(sequence identification, see SEQ ID 4):
wherein N is any of G, C, A or T;
wherein Y is any of C or T;
wherein R is any of A or G;
wherein K is any of G or T;
wherein W is A or T;
wherein B is G, T or C;
wherein D is G, A or T.

In other words, the consensus sequence is as follows:
wherein X at position 2 is any of G or T;
wherein X at position 3 is any of C or T;
wherein X at position 33 is any of A or G;
wherein X at position 46 is any of A or G;
wherein X at position 48 is any of G, C, A or T;
wherein X at position 54 is any of G, A or T;
wherein X at position 55 is any of C or T;
wherein X at position 56 is any of A or G;
wherein X at position 58 is any of G, T or C;
wherein X at position 59 is any of G, A or T;
wherein X at position 61 is any of A or G;
wherein X at position 62 is any of G, C, A or T;
wherein X at position 63 is any of G, C, A or T;
wherein X at position 66 is any of G, C, A or T;
wherein X at position 67 is any of G, C, A or T;
wherein X at position 68 is any of G, A or T;
wherein X at position 69 is any of G, C, A or T;
wherein X at position 70 is any of C or T;
wherein X at position 71 is any of C or T;
wherein X at position 72 is any of G, C, A or T;
wherein X at position 73 is any of A or G;
wherein X at position 75 is any of C or T;
wherein X at position 85 is any of A or T;
wherein X at position 89 is any of C or T;
wherein X at position 97 is any of G, A or T;
wherein X at position 99 is any of C or T;
wherein X at position 114 is any of C or T;
wherein X at position 138 is any of A or G;

SEQ ID 4 identifies the consensus sequence in a certain segment of the 16S rRNA gene which covers at least the genes identified by the respective GenBank accession numbers of the target DNA sequences, as follows:
GenBank accession numbers: AF178677 (SEQ ID 5), AF548631 (SEQ ID 6), AY069948 (SEQ ID 7), AY150972 (SEQ ID 8), AY150976 (SEQ ID 9), AY150977 (SEQ ID 10), AY150984 (SEQ ID 11), AY529632 (SEQ ID 12), DQ157160 (SEQ ID 13), DQ825438 (SEQ ID 14), DQ825441 (SEQ ID 15), DQ825447 (SEQ ID 16), DQ825451 (SEQ ID 17), DQ825453 (SEQ ID 18), DQ825458 (SEQ ID 19), EU888930 (SEQ ID 20), EU442623 (SEQ ID 21), U88563 (SEQ ID 22), EU442616 (SEQ ID 23), EU442617 (SEQ ID 24), EU442618 (SEQ ID 25), EU442619 (SEQ ID 26), EU442620 (SEQ ID 27), EU442621 (SEQ ID 28), EU442622 (SEQ ID 29), EU442624 (SEQ ID 30), EU442625 (SEQ ID 31), EU442626 (SEQ ID 32), EU442627 (SEQ ID 33), EU442628 (SEQ ID 34), EU442630 (SEQ ID 35), EU442631 (SEQ ID 36), EU442632 (SEQ ID 37), EU442633 (SEQ ID 38), EU442634 (SEQ ID 39), EU442635 (SEQ ID 40), EU442636 (SEQ ID 41), EU442637 (SEQ ID 42), EU442638 (SEQ ID 43), EU442639 (SEQ ID 44), EU442640 (SEQ ID 45), U95297 (SEQ ID 46), AF407208 (SEQ ID 47), AY150973 (SEQ ID 48), EF416568 (SEQ ID 49), AF197337 (SEQ ID 50), AY529641 (SEQ ID 51), GQ129115 (SEQ ID 52), GQ129117 (SEQ ID 53), GQ129116 (SEQ ID 54), GQ129118 (SEQ ID 55), GQ129119 (SEQ ID 56), U82963 (SEQ ID 57), GU562823 (SEQ ID 58), AY831867 (SEQ ID 59), EU442629 (SEQ ID 60), DQ157150 (SEQ ID 61), DQ464417 (SEQ ID 62), DQ464421 (SEQ ID 63), DQ464424 (SEQ ID 64), DQ464425 (SEQ ID 65), DQ825448 (SEQ ID 66), DQ825449 (SEQ ID 67), DQ825450 (SEQ ID 68), DQ825454 (SEQ ID 69), EU861063 (SEQ ID 70), AY171918 (SEQ ID 71), FJ667773 (SEQ ID 72), FJ667774 (SEQ ID 73), KC512401 (SEQ ID 74), EF460765 (SEQ ID 75), EF616468 (SEQ ID 76), AB617734 (SEQ ID 77), AB617735 (SEQ ID 78), AB617736 (SEQ ID 79), AB617737 (SEQ ID 80), AB617738 (SEQ ID 81), EU165512 (SEQ ID 82), EU165513 (SEQ ID 83), EU828579 (SEQ ID 84), EU828580 (SEQ ID 85), EU828581 (SEQ ID 86), JF931131 (SEQ ID 87), JF931132 (SEQ ID 88), JF931133 (SEQ ID 89), JF931136 (SEQ ID 90), JF931137 (SEQ ID 91), AF338268 (SEQ ID 92), CP006935 (SEQ ID 93), CP006935 (SEQ ID 94), KF313922 (SEQ ID 95), EU165511 (SEQ ID 96), EU165509 (SEQ ID 97), EU165510 (SEQ ID 98), EU828582 (SEQ ID 99), JF931134 (SEQ ID 100), JF931135 (SEQ ID 101), JF931138 (SEQ ID 102), FJ440328 (SEQ ID 103), EU916726 (SEQ ID 104), GU230142 (SEQ ID 105), GU230143 (SEQ ID 106), GU230144 (SEQ ID 107), HQ197746 (SEQ ID 108), HQ197745 (SEQ ID 109), HQ197747 (SEQ ID 110), HQ197744 (SEQ ID 111), HQ197742 (SEQ ID 112), HQ197743 (SEQ ID 113), HQ197748 (SEQ ID 114), HQ197749 (SEQ ID 115), AB610845 (SEQ ID 116), AB610846 (SEQ ID 117), AB610850 (SEQ ID 118), CP006771 (SEQ ID 119), AF271154 (SEQ ID 120), AY150974 (SEQ ID 121), AY150978 (SEQ ID 122), AY150979 (SEQ ID 123), AY150980 (SEQ ID 124), AY150981 (SEQ ID 125), AY297712 (SEQ ID 126), AM691834 (SEQ ID 127), DQ157149 (SEQ ID 128), DQ825439 (SEQ ID 129), DQ825440 (SEQ ID 130), DQ825442 (SEQ ID 131), DQ825443 (SEQ ID 132), DQ825444 (SEQ ID 133), DQ825445 (SEQ ID 134), DQ825446 (SEQ ID 135), DQ825452 (SEQ ID 136), DQ825455 (SEQ ID 137), DQ825456 (SEQ ID 138), DQ825457 (SEQ ID 139), FJ004275 (SEQ ID 140), U88564 (SEQ ID 141), AY532390 (SEQ ID 142), KF366443 (SEQ ID 143), GQ129113 (SEQ ID 144), GQ129114 (SEQ ID 145), GQ129112 (SEQ ID 146), EF416569 (SEQ ID 147), AF338269 (SEQ ID 148), JQ897388 (SEQ ID 149), GU124610 (SEQ ID 150), AF306346 (SEQ ID 151), JN214359 (SEQ ID 152), JN214357 (SEQ ID 153), JN214358 (SEQ ID 154), JN214356 (SEQ ID 155), FN908079 (SEQ ID 156), FN908077 (SEQ ID 157), FN908080 (SEQ ID 158), FN908081 (SEQ ID 159), FN908082 (SEQ ID 160), FN908084 (SEQ ID 161), FN908083 (SEQ ID 162), FN908078 (SEQ ID 163), FN908076 (SEQ ID 164), JF495171 (SEQ ID 165), FJ527244 (SEQ ID 166), GU047355 (SEQ ID 167), AF029394 (SEQ ID 168), AY492086 (SEQ ID 169), AB610847 (SEQ ID 170), NC_015153 (SEQ ID 171), NC_015155 (SEQ ID 172), HQ259257 (SEQ ID 173), U88565 (SEQ ID 174), AF016546 (SEQ ID 175), AY946266 (SEQ ID 176), EU367964 (SEQ ID 177), CP003703 (SEQ ID 178), DQ641256 (SEQ ID 179), AB558897 (SEQ ID 180), AB558899 (SEQ ID 181).

Specifically, the method described herein covers any and all species of haemoplasmas characterized by the sequences identified as SEQ ID 5 - SEQ ID 180.

It was surprising that such coverage could be obtained by the test described herein despite the variability of the 16S rRNA gene sequences within the haemofelis and haemominutum groups, and between both groups. At the same time, the high specificity of the test ensures the absence of false positives that might occur because of any non-haemotropic mycoplasma contamination.

Specifically, the method described herein further comprises detecting at least one negative control and/or at least one positive control.

Specifically, said detecting at least one negative control comprises amplifying and detecting a nucleic acid sequence of a plasmid not containing the nucleotide sequence identified as SEQ ID 4.

Specifically, said detecting at least one positive control comprises amplifying and detecting a nucleic acid sequence of a plasmid containing the nucleotide sequence identified as SEQ ID 4, and the flanking portions of the polynucleotide ("flanking" meaning: the nucleotide sequences adjacent to the 5' end and the 3' end of SEQ ID 4, respectively) which are complementary to the forward and the reverse primers, and capable of hybridizing with the respective primers under standard condition.

Preferably, the negative control is used in combination with a positive control, wherein the negative control is the same plasmid that is otherwise used to incorporate the positive control haemoplasma 16S rRNA gene or the gene segment encompassing the nucleotide sequence identified as SEQ ID 4 and the flanking portions capable of hybridizing with the forward and reverse primers. An exemplary control plasmid is pBSK (Stratagene), which - when used as a positive control - comprises the haemoplasma 16S rRNA gene or the gene segment, or - when used as a negative control - is the same plasmid, yet without such haemoplasma 16S rRNA gene or the gene segment.

Positive controls can be any 16S rDNA sequence or genomic DNA of any of the haemoplasma species covered by the test described herein. A specific positive control is any of the following haemoplasma 16S rRNA genes cloned into a standard cloning vector e.g., pBSK(+)Simple-Amp (Biomatik): *Mycoplasma haemofelis* (Mhf), *Mycoplasma haemomuris* (Mhmu), *Candidatus* Mycoplasma haemohominis (CMhh), *Candidatus* Mycoplasma turicensis (CMt), *Mycoplasma coccoides* (Mcc), *Mycoplasma ovis* (Movi), *Candidatus* Mycoplasma haemominutum (CMhm), *Candidatus* Mycoplasma haemolamae (CMhla), *Mycoplasma suis* (Msui), or *Mycoplasma wenyonii* (Mwyi).

Specifically, said sample is a sample of mammalian, mammalian cells or *in vitro* cell culture, in particular humans and other primates, including non-human primates such as chimpanzees and other apes and monkey species; farm animals such as cattle, sheep, pigs, goats and horses; domestic mammals such as dogs and cats; laboratory animals including rodents such as mice, rats and guinea pigs; and the like. Specifically, said sample is provided to perform the test described herein *ex vivo.*

Specifically, said sample is a mammalian body fluid selected from the group consisting of blood, blood fractions, plasma, bone marrow, urine, stool, saliva, lymph, exudates, transudates, secretions, spinal fluid, seminal fluid, dispersed tissue and/or fluids from natural or non-natural body cavities or smears, in particular blood smears.

Specifically, said sample is a sample of an *in vitro* cell culture, including e.g. cellular material, cell culture medium, or cell culture products.

Specifically, said sample is obtained from source material and/or in-process control material and/or final control material, in a method of producing pharmaceutical products, e.g. cell culture products such as recombinant proteins, or blood products, or plasma derivatives.

Specifically, said sample is obtained from an animal, including human beings, to diagnose haemoplasma infection and the respective disease.

The invention further provides for a composition comprising an oligonucleotide primer pair consisting of a forward primer comprising at least 10 consecutive bases of SEQ ID 1 and a reverse primer comprising at least 10 consecutive bases of SEQ ID 2.

Specifically, the forward primer comprises or consists of at least any of 10, 11, 12, 13, 14, 15, 16, 17, 18, 19 or 20 bases of SEQ ID 1.

Specifically, the reverse primer comprises or consists of at least any of 10, 11, 12, 13, 14, 15, 16, 17, 18, 19 or 20 bases of SEQ ID 2.

Specifically, the forward primer comprises or consists of SEQ ID 1, and the reverse primer comprises or consists of SEQ ID 2.

Specifically, the composition comprises the primers in the isolated form.

Specifically, the composition is provided in a kit of parts or composite reagent ready for use in a qPCR assay, preferably wherein the primer pair is provided in a mixture, or in separate containments, preferably in a packaged combination.

An exemplary formulation of the primer mix comprises: 3-8 µM forward primer, 5-8 µM µM reverse primer, 2-3 µM dual labeled probe, 1xTris/EDTA buffer (10 mM Tris, 1 mM EDTA (ethylenediaminetetraacetic acid), pH 8.0). This exemplary formulation is equivalent to a 10 x concentrated primer mix that can be diluted to a 1× concentrated primer mix (300-800 nM forward primer, 500-800 nM reverse primer, 200-300 nM dual labeled probe) in the final qPCR reaction mixture.

Specifically, said primer composition or primer mix is provided in a storage stable form, preferably in 1xTris/EDTA buffer or lyophilized. The invention further provides a set or kit of parts in a packaged combination, comprising the composition of the forward and reverse primer as described herein, the positive and/or negative controls (e.g. plasmid controls described herein), e.g., the components in two or more containments.

Specifically, as a positive control, a linear standard cloning plasmid (e.g. a linear pBSK vector) is provided containing a complete or nearly complete 16S rRNA gene of any of the haemoplasma species covered by the test described herein. Plasmids can be provided at various concentrations in buffer, e.g. 1xTris/EDTA buffer. Specifically, as a negative control in combination with the above mentioned positive control, the same linear standard cloning plasmid is used, yet without the 16S rRNA gene that is otherwise used in the positive control.

Specifically, the set or kit of parts comprises at least a dsDNA dye, such as SYBR® Green dye, a reference dye, a template dependent nucleic acid extending enzyme such as a Hot Start Taq polymerase that has been either chemically inhibited or antibody inactivated to avoid non-specific amplification of the polymerase at lower temperatures.

Specifically, the set or kit of parts further comprises a solution for optimal function of the nucleic acid extending enzyme.

Specifically, the set or kit of parts further comprises deoxyribonucleotide triphosphates.

### FIGURES

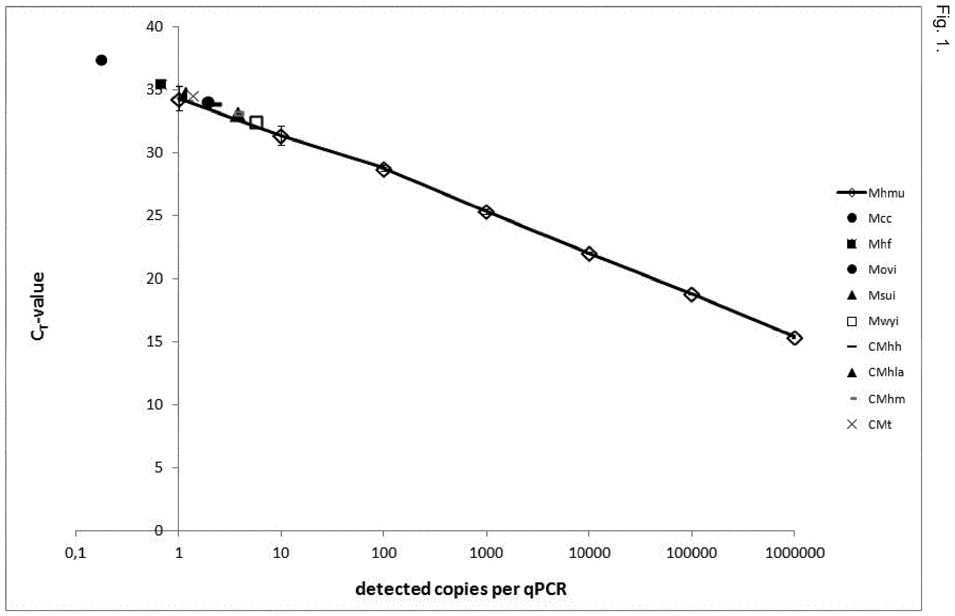

Figure 1: Sensitivity of the generic haemoplasma qPCR assay.
   Scatter plot with standard curve for qPCR amplification of the 16S rRNA gene of *M*. *haemomuris* (Mhmu) target sequence and amplification of target sequences from 9 additional control plasmids: Mcc (*Mycoplasma coccoides*)*,* Mhf (*Mycoplasma haemofelis*)*,* Movi (*Mycoplasma ovis*)*,* Msui (*M. suis*)*,* Mwyi (*Mycoplasma wenyonii*)*,* CMhh (*Candidatus* Mycoplasma haemohominis), CMhla (*Candidatus* Mycoplasma haemolamae), CMhm (*Candidatus* Mycoplasma haemominutum), CMt (*Candidatus* Mycoplasma turicensis).
Figure 2: Target sequence amplification from genomic DNA of *M*. *suis.*
   Amplification curves of the target sequence of the 16S rRNA gene. Analysis of different dilutions of the Mhmu positive control and genomic DNA of *M. suis* isolated from porcine blood.
Figure 3: Sensitivity of the generic haemoplasma qPCR in blood samples spiked with the Mhmu control plasmid. Amplification of the target sequence from three whole blood samples experimentally spiked with Mhmu control plasmid at concentrations ranging from 10 to 10⁶ copies per ml blood.

### DETAILED DESCRIPTION

Specific terms as used throughout the specification have the following meaning. The term "real-time PCR" or "qPCR" as used herein is understood as follows:
"Polymerase Chain Reaction" or "PCR" refers to a nucleic acid-based test (NAT) or nucleic acid amplification test (NAAT), which involves amplifying a specific target polynucleotide through repeated cycles of denaturation of double stranded DNA, annealing of oligonucleotide primers, and DNA polymerization using a thermostable DNA polymerase in the presence of nucleotide triphosphates. As used herein, "qPCR" refers to a PCR reaction performed in such a way and under such controlled conditions that the results of the assay are quantitative; i.e., the assay is capable of quantifying the amount of target polynucleotide present in the sample.

When the template or target nucleic acid is RNA, a reverse transcriptase polymerase chain reaction or "RT-PCR" may be performed. Such method refers to a variant of the basic PCR method wherein the starting material is an RNA, which is reverse transcribed into a cDNA prior to PCR. "RT-qPCR" refers to RT-PCR performed under conditions that afford quantitation of the RNA present in the sample.

As described herein, the primers and optionally probes can be used in PCR-based techniques, to detect the presence of haemoplasmas in biological samples. Such techniques provide for amplifying a desired target nucleic acid sequence contained in a nucleic acid molecule or mixture of molecules. A pair of primers is employed in excess to hybridize to the complementary strands of the target nucleic acid. The primers are each extended by a polymerase using the target nucleic acid as a template. The extension products become target sequences themselves after dissociation from the original target strand. New primers are then hybridized and extended by a polymerase, and the cycle is repeated to geometrically increase the number of target sequence molecules. PCR methods for amplifying target nucleic acid sequences in a sample are well-known in the art and have been described in, e.g., Innis et al. (eds.) PCR Protocols (Academic Press, NY 1990); Taylor (1991) Polymerase chain reaction: basic principles and automation, in PCR: A Practical Approach, McPherson et al. (eds.) IRL Press, Oxford.

The qPCR method described herein relies on the broad coverage and high specificity of the detection method. The test is specifically performed in a manner to detect low levels of a specific target haemoplasma polynucleotide present in a sample.

Any sample that is suspected of containing any haemoplasma bacterium may be tested in the method. The person skilled in the art will appreciate that certain samples may require pretreatments, such as dilution, buffering, extraction, filtration and the like, prior to carrying out the method described herein. For the detection of haemoplasmas, preferred are samples derived from biological materials including animal derived materials such as blood and blood products, sera, tissues, tissue extracts, cells and cellular extracts, and materials given off or otherwise excreted or eliminated by an animal or cell in a cell culture. These materials may include clinical specimens, as well as raw materials and supplies used in industrial or other processes that require the exclusion of haemoplasma contamination. Specific samples are those comprising preparations of biological materials in various states of purity as may be derived from in-process sampling during preparation and purification.

Quantitation of the specific target polynucleotide is accomplished by performing PCR under appropriate conditions and measuring, either directly or indirectly, the production of amplified copies of the target polynucleotide. An especially useful method to quantify the target polynucleotide is by use of the TaqMan® technique (PE Biosystems, Foster City, USA). Any method that allows detection and quantitation of amplified products that are produced as a result of performing PCR on a specific polynucleotide target is suitable for use in the test described herein. Examples of such methods include quantitative competitive PCR or PCR followed by gel electrophoresis with direct quantitation of the amplicon band in the gel by densitometry.

The term "primer" or "oligonucleotide primer" as used herein, refers to an oligonucleotide that hybridizes to the template strand of a nucleic acid and serves as an initiation point for synthesis of a nucleic acid strand complementary to the template strand when placed under conditions in which synthesis of a primer extension product is induced, i.e., in the presence of nucleotides and a polymerization-inducing agent such as a DNA or RNA polymerase and at suitable temperature, pH, metal concentration, and salt concentration. The primer is single-stranded for maximum efficiency in amplification, but may alternatively be double-stranded. If double-stranded, the primer can first be treated to separate its strands before being used to prepare extension products. This denaturation step is typically effected by heat, but may alternatively be carried out using alkali, followed by neutralization. Thus, a "primer" is complementary to a template, and complexes by hydrogen bonding or hybridization with the template to give a primer/template complex for initiation of synthesis by a polymerase, which is extended by the addition of covalently bonded bases linked at its 3' end complementary to the template in the process of DNA or RNA synthesis.

As described herein, haemoplasma nucleic acids are amplified using a set of oligonucleotide primers comprising the "forward" primer and the "reverse" primer capable of hybridizing to regions of the 16S rRNA gene or its cDNA flanking the portion of the polynucleotide to be amplified. A forward primer is in the 5' to 3' orientation complementary to the haemoplasma sense genomic DNA strand. A reverse primer is in the reverse complement orientation and complementary to the antigenomic haemoplasma template produced during replication or amplification of haemoplasma nucleic acids.

The term "amplicon" refers to the amplified nucleic acid product of a PCR reaction. Amplicons as described herein comprises DNA generated by PCR or RT-PCR.

As used herein, the term "probe" or "oligonucleotide probe" refers to a polynucleotide, that contains a nucleic acid sequence complementary to a nucleic acid sequence present in the target nucleic acid analyte or the amplicon produced by amplification of the target, capable of hybridizing with such analyte and amplicon, respectively under standard assay conditions.

The polynucleotide regions of probes used herein may be composed of DNA and/or synthetic nucleotide analogs. Probes may be labeled in order to detect the target sequence. Such a label may be present at the 5' end, at the 3' end, at both the 5' and 3' ends, and/or internally. For example, when a probe is to be used in a fluorogenic 5' nuclease assay, such as used in the TaqMan™ technique, the probe will typically contain at least one fluorescer and at least one quencher which is removed by the endonuclease or exonuclease activity of a polymerase used in the reaction in order to detect any amplified target oligonucleotide sequences. In this context, the oligonucleotide probe will have a sufficient number of phosphodiester linkages adjacent to its 5' end so that the 5' to 3' nuclease activity employed can efficiently degrade the bound probe to separate the fluorescers and quenchers. Additionally, the oligonucleotide probe will typically be derived from a sequence that lies between the regions of the target hybridizing to the forward and the reverse primers when used in a 5' nuclease assay.

It will be appreciated that the hybridizing sequences need not have 100% complementarity to provide stable hybrids. In some situations, stable hybrids will form where fewer than about 10% of the bases are mismatches, ignoring loops of four or more nucleotides. Accordingly, as used herein hybridization of primers or a probe to the respective target nucleotide sequence requires "complementarity", which term refers to an oligonucleotide that forms a stable duplex with its "complement" under assay conditions, generally where there is about 90% or greater homology.

Specific embodiments refer to naturally-occurring nucleotide sequences, yet in an artificial environment and assay system. Thus, oligonucleotides or gene sequences are provided in the isolated form or as artificial products, and amplicons are synthetic products resulting from amplification by PCR. Typically, the oligonucleotides are provided as synthetic products or compositions, thus are isolated, i.e. sufficiently separated from other nucleic acids with which they would be associated in its natural state, or separated from other components present during its synthesis.

The consensus sequence (SEQ ID 4) described herein is an artificial sequence. Therefore, determining such consensus sequence employing the primer pair (oligonucleotides) and method described herein does involve artificial materials, and the claims refer to significantly more than just describing a natural relationship.

The foregoing description will be more fully understood with reference to the following examples. Such examples are, however, merely representative of methods of practicing one or more embodiments of the present invention and should not be read as limiting the scope of invention.

### EXAMPLES

### Methods:

### Isolation of genomic DNA from samples

DNA purification from samples can either be performed manually or automated and can contain any of the following treatment steps: cell lysis, protein digestion, protein and membrane precipitation, DNA binding to silica resin or DNA-binding beads, DNA precipitation, DNA washing and DNA elution or resuspension. The DNA is preferably isolated with a commercially available DNA extraction kit.

Manual DNA extraction using spin columns with silica resin includes the following steps: Cell lysis, proteinase K digestion, RNase A digestion, DNA binding to silica resin, washing and elution.

### Exemplary Kits:

QIAamp® DNA Blood Mini Kit (QIAGEN): The kit "provides silica-membrane-based DNA purification. Mini kits are designed for processing up to 200µl of human whole blood, and DNA is eluted in a final volume of 50-200µl."

GenElute™ Mammalian Genomic DNA Miniprep Kit (Sigma-Aldrich): The kit "provides a simple and convenient way to isolate pure genomic DNA from a variety of cultured cells, tissues (including rodent tails), and fresh whole blood or white blood cells. The kit combines the advantages of silica binding with a microspin format and eliminates the need for expensive resins, alcohol precipitation, and hazardous organic compounds such as phenol and chloroform. The starting materials are lysed in a chaotropic salt-containing solution to insure the thorough denaturation of macromolecules. Addition of ethanol causes DNA to bind when the lysate is spun through a silica membrane in a microcentrifuge tube. After washing to remove contaminants, DNA is eluted in 200 µL of a Tris-EDTA solution."

Manual or automated DNA extraction using DNA-binding beads includes the following steps: Cell lysis, proteinase K digestion, RNase A digestion, DNA binding to beads, washing and elution.

### Exemplary Kits:

Maxwell@ HT 96 gDNA Blood Isolation System (Promega)
MagNA Pure 96 DNA and Viral NA Small Volume Kit and MagNA Pure 96 instrument (Roche)

### PCR

The polymerase chain reaction involves the use of the pair of specific oligonucleotide primers (SEQ ID 1 and SEQ ID 2) to initiate DNA synthesis on a target DNA template. Two oligonucleotide primers are used for each double-stranded sequence to be amplified. The target sequence is heat denatured into its complimentary strands. Each primer, which is sufficiently complementary to a region of each strand of the target sequence to hybridize with it, anneals to one of the strands by decreasing the reaction temperature. The primers are extended, using nucleosides in the sample and a heat stable polymerization agent of the DNA deoxynucleotidyltransferase EC 2.7.7.7. Examples of such heat stable polymerases are *Taq* DNA polymerase deriving from *Thermus aquaticus, Tgo* DNA polymerase from *Thermococcus gorgonarius, Pfu* DNA polymerase from *Pyrococcus furiosus,* Pfx and processivity-enhanced enzymes thereof). Primer extension occurs by increasing the temperature in the reaction mixture to an optimal extension temperature for the DNA polymerase. The primer extension products hybridize to the complementary strand of the target sequence. The amplification products are then separated from the template strands, and the process is repeated until the desired level of amplification is obtained. In subsequent cycles, the primer extension products serve as new templates for synthesizing the targeted nucleic acid sequence.

### Monitoring the targeted nucleic acid sequence

The amplified DNA is detected either by standard techniques such as gel electrophoresis or hybridization with a target specific probe or by real time qPCR analysis using DNA intercalating dyes or a double labeled fluorogenic probe.

By incorporating a detectable entity into the primers SEQ ID 1 or SEQ ID 2, the amplified DNA can be monitored by gel electrophoresis. A detectable entity can be a detectable molecule or compound, such as a fluorophore or radioactive isotype. The amplified PCR product can also be visualized in a gel by using a DNA intercalating molecule such as ethidium bromide.

When a probe is used, the amplified DNA is denatured into it complementary single strands. The probe is brought into contact with a mixture containing the denatured amplified target nucleic acid sequence. A determination is made whether or not hybridization has occurred. Preferably, the probe is an oligonucleotide such as SEQ ID 3 that has been labeled with a detectible entity such as fluorescently or radioactive labeled molecules.

One method for monitoring real-time or endpoint accumulation of the amplicon is with the use of a dsDNA intercalating fluorescent dye (exemplary agents include SYBR GREEN™, SYBR GOLD™, and EVAGREEN™) in the PCR reaction mixture. The signal of the intercalating dye increases with the increase in double stranded nucleic acids.

Another method for detection of amplification products is the 5'-3' exonuclease "hydrolysis" PCR assay (also referred to as the TaqMan™ assay, e.g. Holland et al. Proc Natl Acad Sci USA. 1991, 88(16): 7276-7280).

The TaqMan technique and probe principle relies on the 5'-3' exonuclease activity of a Taq polymerase to cleave a dual-labeled probe during hybridization to the complementary target sequence and fluorophore-based detection. As in other quantitative PCR methods, the resulting fluorescence signal permits quantitative measurements of the accumulation of the product during the exponential stages of the PCR. In principle, the TaqMan™ assay detects the accumulation of a specific PCR product by hybridization and cleavage of a double labeled fluorogenic probe during the amplification reaction. The fluorogenic probe may consists of an oligonucleotide (e.g. SEQ ID 3) labeled with both a fluorescent reporter dye and a quencher dye. During PCR, this probe is cleaved by the 5'-exonuclease activity of DNA polymerase if, and only if, it hybridizes to the segment being amplified. Cleavage of the probe generates an increase in the fluorescence intensity of the reporter dye.

### Positive and negative control plasmids

Positive controls are linearized pBSK containing the nearly complete 16S rRNA gene of any of the species covered by the test described herein, either lyophilized or in 1x TE buffer. The negative control is a linearized pBSK either lyophilized or in 1x TE buffer.

*Escherichia coli* was transformed with a plasmid vector containing a nearly complete haemoplasma 16S rRNA gene or with the plasmid devoid of the 16S rDNA sequence. The plasmids were re-isolated from *E. coli* and were linearized by endonuclease digestion. After a further purification step, plasmid DNA was quantified photometrically. Dilutions of the linearized plasmid serve as standard controls in the qPCR analysis for copy number quantification.

### Example 1: DNA amplification of template (control plasmids) using the SYBR Green PCR protocol

To assess the sensitivity of the qPCR assay and to determine whether 1 DNA copy can still be detected by the generic haemoplasma qPCR, a plasmid containing the 16S rRNA gene of *M*. *haemomuris* (Mhmu) was diluted and applied at various concentrations to generate a standard curve (Fig. 1). The reaction mixture was composed of 12.5 µl of 2x PowerUp™ SYBR® Green Master Mix (ThermoFisher Scientific), 300 nM forward primer, 800 nM reverse primer and 10 µl of DNA sample. Assays were run on a 7500 Fast Real-Time PCR System (ThermoFisher Scientific). The PCR protocol was as follows: 50°C for 2 min and 95°C for 2 min, followed by 40 cycles of 95°C for 5 s and 60°C for 30 sec. After the PCR run, dissociation was performed with the following thermal settings: 95°C for 15 s, 60°C for 60 s and finally 95°C for 15 s. The C_{T} threshold was set at 0.2. In six replicates, the 1 copy per PCR reaction was detectable (Fig. 1, Table 1).

To assess whether all positive controls can be detected with the generic haemoplasma qPCR assay and to determine the sensitivity of the assay, dilutions of all positive controls were analyzed. All positive control plasmids were detected at concentrations ranging from 0.2 to 6 copies per PCR reaction (Fig.1, Table 1), therefore the sensitivity of the assay is < 1 copy of 16S rDNA per PCR reaction.

### Example 2: DNA Amplification of template (control plasmid and genomic DNA) by SYBR Green PCR

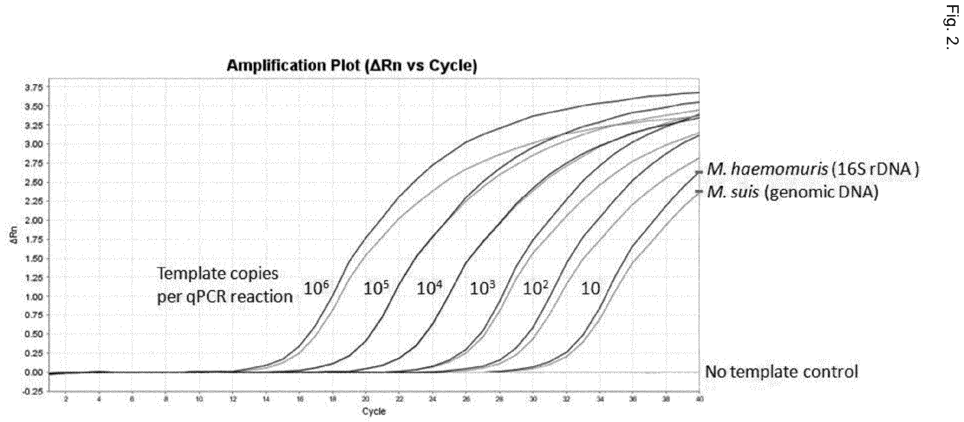

To assess whether genomic haemoplasma DNA can be detected with the generic haemoplasma qPCR assay, the genomic DNA of *M*. *suis* KI3806 (Oehlerking et al. 2011, J Bacteriol. 193: 2369-2370).was analyzed at various concentrations (Fig. 2). DNA of a *M*. *suis* infected blood was isolated. To reduce background DNA from erythrocytes, the whole blood was first diluted 1:80 in porcine serum. DNA was isolated from 1 ml diluted sample with the GenElute™ Mammalian Genomic DNA Miniprep Kit (Sigma-Aldrich) and eluted with 200 µl elution buffer. The eluted DNA was quantified with the NanoDrop 3300 Fluorospectrometer (ThermoFisher Scientific). Dilutions of the *M*. *suis* DNA were quantified using the generic haemoplasma qPCR assay and the Mhmu positive control plasmid as reference (Fig. 2). PCR reaction mixtures and settings were the same as in Example 1. Amplifications for diluted genome copies had similar C_{T}-values as the diluted control plasmid.

### Example 3: DNA amplification of template (control plasmids) from experimentally spiked blood samples using the SYBR Green PCR protocol

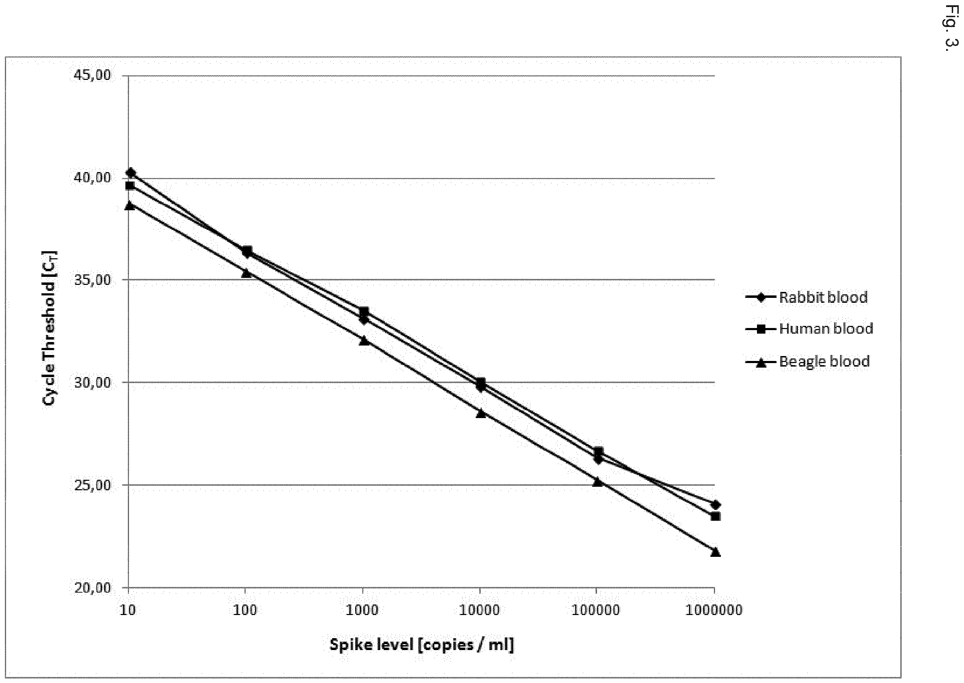

To assess whether the PCR assay is sensitive enough to detect 10 copies per ml of blood, three product matrices (rabbit whole blood, human whole blood and beagle whole blood) were experimentally spiked with different concentrations of the Mhmu control plasmid. Spike levels ranged from 10 to 10⁶ copies per ml product matrix. DNA was extracted with the MagNA Pure 96 instrument (Roche) from 1 ml of each spiked or unspiked sample and eluted with 100 µl DNA extraction buffer. PCR reaction mixtures and settings were the same as in Example 1, except that 48 cycles were run. In all three product matrices, the 10 copies per ml spike level could be detected (Fig. 3, Table 2), meaning that the PCR has a detection-sensitivity between 1 and 10 copies per PCR reaction or 10 copies per ml whole blood sample. No cross reaction was detected with DNA from the unspiked product matrices.

### Example 4: Specificity and selectivity of haemoplasma detection by the generic qPCR assay

To assess whether the generic haemoplasma PCR assay cross reacts with DNA from related mycoplasma species, genomic DNA from different mycoplasma species at a concentration of 10⁶ genome copies per reaction was analyzed. DNA was isolated from liquid cultures with the GenElute™ Mammalian Genomic DNA Miniprep Kit (Sigma-Aldrich). The eluted DNA was quantified with the NanoDrop 3300 Fluorospectrometer (ThermoFisher Scientific). DNA concentrations usually ranged from 10⁶-10⁷ genome copies per µl eluate. DNA was diluted in 1 x TE to 10⁵ genome copies per µl and per PCR reaction, 10 µl was applied. PCR reaction mixtures and settings were the same as in Example 1, except that 48 cycles were run. Amplification results were determined by comparison to a standard curve of the Mhmu control plasmid (Table 3).

### TABLES

**Table 1. Sensitivity of the generic haemoplasma qPCR assay based on the number of detected copies of control plasmids**

| Mycoplasma species | Control plasmid | C_{T}-value | Copies per qPCR reaction |
|---|---|---|---|
| *Mycoplasma haemomuris* | Mhmu | 15,44* | 1000000 |
| | | 18,86* | 100000 |
| | | 22,10* | 10000 |
| | | 25,38* | 1000 |
| | | 28,82* | 100 |
| | | 31,42* | 10 |
| | | 34,36* | 1 |
| *Mycoplasma coccoides,* | Mcc | 34,04 | 1,95 |
| *Mycoplasma haemofelis* | Mhf | 35,53 | 0,68 |
| *Mycoplasma ovis* | Movi | 37,41 | 0,18 |
| *Mycoplasma suis* | Msui | 34,75 | 1,18 |
| *Mycoplasma wenyonii* | Mwyi | 32,49 | 5,83 |
| *Candidatus* Mycoplasma haemohominis | CMhh | 33,88 | 2,18 |
| Candidatus *Mycoplasma haemolamae* | CMhla | 33,12 | 3,74 |
| *Candidatus* Mycoplasma haemominutum | CMhm | 33,16 | 3,63 |
| *Candidatus* Mycoplasma turicensis | CMt | 34,53 | 1,37 |

| | | | |
|---|---|---|---|
| *: average of 6 replicates | | | |

**Table 2. Sensitivity of the generic haemoplasma qPCR assay based on C_{T}-values of spiked and unspiked test samples**

| Spike level (plasmid copies per ml sample) | Whole blood | | |
|---|---|---|---|
| | Rabbit | Human | Beagle |
| unspiked | - | - | - |
| 10 | 40.30 | 39.63 | 38.74 |
| 100 | 36.38 | 36.47 | 35.44 |
| 1000 | 33.18 | 33.55 | 32.16 |
| 10000 | 29.85 | 30.03 | 28.61 |
| 100000 | 26.36 | 26.68 | 25.26 |
| 1000000 | 24.13 | 23.53 | 21.84 |

| | | | |
|---|---|---|---|
| -: negative | | | |

**Table 3. Specificity of the generic haemoplasma qPCR assay based on the lack of detection of related mycoplasma species**

| Mycoplasma species | Detected genome copies out of 10⁶ genome copies per qPCR reaction |
|---|---|
| *Acholeplasma laidlawii* | - |
| *Mycoplasma arginini* | - |
| *Mycoplasma bovigenitalium* | - |
| *Mycoplasma bovirhinis* | - |
| *Mycoplasma bovoculi* | - |
| *Mycoplasma californicum* | - |
| *Mycoplasma canis* | - |
| *Mycoplasma canadense* | - |
| *Mycoplasma cynos* | - |
| *Mycoplasma dispar* | 0.58 |
| *Mycoplasma edwardii* | - |
| *Mycoplasma fastidiosum* | 0.25 |
| *Mycoplasma felis* | - |
| *Mycoplasma fermentans* | - |
| *Mycoplasma gallisepticum* | - |
| *Mycoplasma gateae* | - |
| *Mycoplasma hominis* | - |
| *Mycoplasma hyopneumoniae* | - |
| *Mycoplasma hyorhinis* | 0.28 |
| *Mycoplasma maculosum* | - |
| *Mycoplasma molare* | - |
| *Mycoplasma mucosicanis* | - |
| *Mycoplasma mycoides mycoides SC* | 0.02 |
| *Mycoplasma orale* | - |
| *Mycoplasma pneumoniae* | - |
| *Mycoplasma salivarium* | - |
| *Mycoplasma synoviae* | - |
| *Spiroplasma citri* | - |

| | |
|---|---|
| -: negative | |

## Claims

1. A method for detecting at least one haemoplasma species within the group of haemoplasmas of the genus *Mycoplasma* in a biological sample, said method comprising the steps of:
i) treating said sample to release haemoplasma nucleic acid in the sample,
ii) amplifying a segment of a 16S rRNA gene by real-time PCR (qPCR) using an oligonucleotide primer pair, said oligonucleotide primer pair consisting of a forward primer comprising at least 10 consecutive bases of SEQ ID 1 and a reverse primer comprising at least 10 consecutive bases of SEQ ID 2, thereby producing an amplification product, and
iii) detecting the amplification product by means producing a detection signal correlating with the amount of the amplification product which is indicative of a haemoplasma species within the genus *Mycoplasma.*

2. The method of claim 1, wherein the forward primer comprises or consists of SEQ ID 1, and the reverse primer comprises or consists of SEQ ID 2.

3. The method of claim 1 or 2, wherein the amplification product is indicative of a group of different haemoplasma species at least comprising *Mycoplasma haemofelis, Mycoplasma haemocanis, Mycoplasma haemomuris, Candidatus* Mycoplasma haemohominis, *Candidatus* Mycoplasma turicensis, *Mycoplasma coccoides, Candidatus* Mycoplasma haemomacaque, *Candidatus* Mycoplasma haemobos, *Candidatus* Mycoplasma haemovis, *Mycoplasma ovis, Mycoplasma parvum, Candidatus* Mycoplasma haemominutum, *Candidatus* Mycoplasma haematoparvum, *Candidatus* Mycoplasma kahanei, *Candidatus* Mycoplasma haemozalophi, *Candidatus* Mycoplasma haemolamae, *Mycoplasma suis, Candidatus* Mycoplasma haemodidelphis, *Mycoplasma wenyonii, Candidatus* Mycoplasma erythrocervae, and *Candidatus* Mycoplasma haemocervae.

4. The method of any of claims 1 to 3, wherein the amplification product is detected by a quantitative detection method.

5. The method of claim 4, wherein the quantitative detection method employs
i) a double-stranded DNA-binding dye (dsDNA dye) as reporter, preferably wherein the dsDNA dye is fluorescent and the amplification product is detected by measuring fluorescence; or
ii) at least one probe specifically annealing with the amplification product and comprising a detectable label, preferably wherein said at least one probe is an oligonucleotide consisting of SEQ ID 3.

6. The method of any of claims 1 to 5, wherein said treating step i) comprises lysing bacterial cells, nucleic acid extraction and purification, thereby obtaining purified bacterial nucleic acid in a buffer.

7. The method of any of claims 1 to 6, wherein said segment of the 16S rRNA gene is part of a cDNA obtained by reverse transcription of RNA into cDNA using a reverse transcriptase.

8. The method of any of claims 1 to 7, wherein said segment of the 16S rRNA gene comprises the nucleotide sequence identified as SEQ ID 4.

9. The method of any of claims 1 to 8, further comprising detecting, at least one negative control and/or at least one positive control.

10. The method of claim 9, wherein said detecting at least one negative control comprises amplifying and detecting a nucleic acid sequence of a plasmid not containing the nucleotide sequence identified as SEQ ID 4.

11. The method of claim 9 or 10, wherein said detecting at least one positive control comprises amplifying and detecting a nucleic acid sequence of a plasmid containing the nucleotide sequence identified as SEQ ID 4.

12. The method of any of claims 1 to 11, wherein said sample is a mammalian body fluid selected from the group consisting of blood, blood fractions, plasma, bone marrow, urine, stool, saliva, lymph, exudates, transudates, secretions, spinal fluid, seminal fluid, dispersed tissue and/or fluids from natural or non-natural body cavities or smears, or a sample of a cell culture.

13. A composition comprising an oligonucleotide primer pair consisting of a forward primer comprising at least 10 consecutive bases of SEQ ID 1 and a reverse primer comprising at least 10 consecutive bases of SEQ ID 2.

14. The composition of claim 13, wherein the forward primer consists of SEQ ID 1, and the reverse primer consists of SEQ ID 2.

15. The composition of claim 13 or 14, which is provided in a kit of parts or composite reagent ready for use in a qPCR assay, preferably wherein the primer pair is provided in a mixture.

## Patentansprüche

1. Verfahren zum Nachweis mindestens einer Haemoplasma-Spezies innerhalb der Gruppe von Haemoplasmen der Gattung *Mycoplasma* in einer biologischen Probe, wobei das Verfahren die folgenden Schritte umfasst:
i) Behandeln der Probe, um Haemoplasma-Nukleinsäure in der Probe freizusetzen,
ii) Amplifizieren eines Segments eines 16S-rRNA-Gens durch Echtzeit-PCR (qPCR) unter Verwendung eines Oligonukleotid-Primerpaars, wobei das Oligonukleotid-Primerpaar aus einem Vorwärtsprimer umfassend mindestens 10 aufeinanderfolgende Basen von SEQ ID 1 und einem Rückwärtsprimer umfassend mindestens 10 aufeinanderfolgende Basen von SEQ ID 2 besteht, wodurch ein Amplifikationsprodukt produziert wird, und
iii) Nachweisen des Amplifikationsprodukts durch Mittel, die ein Nachweissignal produzieren, das mit der Menge des Amplifikationsprodukts korreliert, was einen Hinweis auf eine Haemoplasma-Spezies innerhalb der Gattung *Mycoplasma* darstellt.

2. Verfahren nach Anspruch 1, wobei der Vorwärtsprimer SEQ ID 1 umfasst oder daraus besteht und der Rückwärtsprimer SEQ ID 2 umfasst oder daraus besteht.

3. Verfahren nach Anspruch 1 oder 2, wobei das Amplifikationsprodukt auf eine Gruppe von unterschiedlichen Haemoplasma-Spezies hinweist, die mindestens *Mycoplasma haemofelis, Mycoplasma haemocanis, Mycoplasma haemomuris, Candidatus* Mycoplasma haemohominis, *Candidatus* Mycoplasma turicensis, *Mycoplasma coccoides, Candidatus* Mycoplasma haemomacaque, *Candidatus* Mycoplasma haemobos, *Candidatus* Mycoplasma haemovis, *Mycoplasma ovis, Mycoplasma parvum, Candidatus* Mycoplasma haemominutum, *Candidatus* Mycoplasma haematoparvum, *Candidatus* Mycoplasma kahanei, *Candidatus* Mycoplasma haemozalophi, *Candidatus* Mycoplasma haemolamae, *Mycoplasma suis, Candidatus* Mycoplasma haemodidelphis, *Mycoplasma wenyonii, Candidatus* Mycoplasma erythrocervae und *Candidatus* Mycoplasma haemocervae umfasst.

4. Verfahren nach einem der Ansprüche 1 bis 3, wobei das Amplifikationsprodukt durch ein quantitatives Nachweisverfahren nachgewiesen wird.

5. Verfahren nach Anspruch 4, wobei das quantitative Nachweisverfahren einsetzt
i) einen an doppelsträngige DNA bindenden Farbstoff (dsDNA-Farbstoff) als Reporter, wobei der dsDNA-Farbstoff vorzugsweise fluoreszierend ist und das Amplifikationsprodukt durch Messen der Fluoreszenz nachgewiesen wird; oder
ii) mindestens eine Sonde, die im Speziellen mit dem Amplifikationsprodukt verschmilzt und einen nachweisbaren Marker umfasst, wobei die mindestens eine Sonde vorzugsweise ein Oligonukleotid bestehend aus SEQ ID 3 ist.

6. Verfahren nach einem der Ansprüche 1 bis 5, wobei der Behandlungsschritt i) Lysieren von Bakterienzellen, Extraktion und Aufreinigung von Nukleinsäure, und dadurch Erhalten von aufgereinigter bakterieller Nukleinsäure in einem Puffer umfasst.

7. Verfahren nach einem der Ansprüche 1 bis 6, wobei das Segment des 16S-rRNA-Gens Teil einer cDNA ist, die durch reverse Transkription von RNA in cDNA unter Verwendung einer reversen Transkriptase erhalten wird.

8. Verfahren nach einem der Ansprüche 1 bis 7, wobei das Segment des 16S-rRNA-Gens die Nukleotidsequenz umfasst, die als SEQ ID 4 identifiziert ist.

9. Verfahren nach einem der Ansprüche 1 bis 8, ferner umfassend das Nachweisen mindestens einer negativen Kontrolle und/oder mindestens einer positiven Kontrolle.

10. Verfahren nach Anspruch 9, wobei das Nachweisen mindestens einer negativen Kontrolle das Amplifizieren und Nachweisen einer Nukleinsäuresequenz eines Plasmids umfasst, das nicht die Nukleotidsequenz enthält, die als SEQ ID 4 identifiziert ist.

11. Verfahren nach Anspruch 9 oder 10, wobei das Nachweisen mindestens einer positiven Kontrolle das Amplifizieren und Nachweisen einer Nukleinsäuresequenz eines Plasmids umfasst, das die Nukleotidsequenz enthält, die als SEQ ID 10 identifiziert ist.

12. Verfahren nach einem der Ansprüche 1 bis 11, wobei die Probe ein Körperfluid vom Säugetier ist, das aus der Gruppe ausgewählt ist, die aus Blut, Blutfraktionen, Plasma, Knochenmark, Urin, Stuhl, Speichel, Lymphe, Exudaten, Transsudaten, Sekreten, spinalem Fluid, Samenflüssigkeit, disgergiertem Gewebe und/oder Fluiden aus natürlichen oder nicht natürlichen Körperhohlräumen oder Abstrichen besteht, oder eine Probe aus einer Zellkultur.

13. Zusammensetzung, umfassend ein Oligonukleotid-Primerpaar bestehend aus einem Vorwärtsprimer, umfassend mindestens 10 aufeinanderfolgende Basen von SEQ ID 1, und einem Rückwärtsprimer, umfassend mindestens 10 aufeinanderfolgende Basen von SEQ ID 2.

14. Verfahren nach Anspruch 13, wobei der Vorwärtsprimer aus SEQ ID 1 besteht und der Rückwärtsprimer aus SEQ ID 2 besteht.

15. Zusammensetzung nach Anspruch 13 oder 14, die in einem Kit aus Teilen oder einem zusammengesetzten Reagens, das gebrauchsfertig in einem qPCR-Assay ist, bereitgestellt wird, wobei das Primerpaar vorzugsweise in einem Gemisch bereitgestellt wird.

## Revendications

1. Procédé de détection d'au moins une espèce d'hémoplasme au sein du groupe des hémoplasmes du genre *Mycoplasma* dans un échantillon biologique, ledit procédé comprenant les étapes de :
i) traitement dudit échantillon pour libérer un acide nucléique d'hémoplasme dans l'échantillon,
ii) amplification d'un segment d'un gène d'ARNr 16S par PCR en temps réel (qPCR) à l'aide d'une paire d'amorces oligonucléotidiques, ladite paire d'amorces oligonucléotidiques étant constituée d'une amorce sens comprenant au moins 10 bases consécutives de SEQ ID 1 et une amorce antisens comprenant au moins 10 bases consécutives de SEQ ID 2, ce qui permet de produire un produit d'amplification, et
iii) détection du produit d'amplification au moyen de la production d'un signal de détection en corrélation avec la quantité du produit d'amplification qui indique une espèce d'hémoplasme au sein du genre *Mycoplasma.*

2. Procédé selon la revendication 1, dans lequel l'amorce sens comprend ou est constituée de SEQ ID 1 et l'amorce antisens comprend ou est constituée de SEQ ID 2.

3. Procédé selon la revendication 1 ou 2, dans lequel le produit d'amplification indique un groupe de différentes espèces d'hémoplasmes comprenant au moins *Mycoplasma haemofelis, Mycoplasma haemocanis, Mycoplasma haemomuris, Candidatus* Mycoplasma haemohominis, *Candidatus* Mycoplasma turicensis, *Mycoplasma coccoides, Candidatus* Mycoplasma haemomacaque, *Candidatus* Mycoplasma haemobos, *Candidatus* Mycoplasma haemovis, *Mycoplasma ovis, Mycoplasma parvum, Candidatus* Mycoplasma haemominutum, *Candidatus* Mycoplasma haematoparvum, *Candidatus* Mycoplasma kahanei, *Candidatus Mycoplasma* haemozalophi, *Candidatus* Mycoplasma haemolamae, *Mycoplasma suis, Candidatus* Mycoplasma haemodidelphis, *Mycoplasma wenyonii, Candidatus* Mycoplasma erythrocervae et *Candidatus* Mycoplasma haemocervae.

4. Procédé selon l'une quelconque des revendications 1 à 3, dans lequel le produit d'amplification est détecté par un procédé de détection quantitative.

5. Procédé selon la revendication 4, dans lequel le procédé de détection quantitative emploie
i) un colorant de liaison à l'ADN double brin (colorant d'ADNdb) comme rapporteur, de préférence le colorant d'ADNdb étant fluorescent et le produit d'amplification étant détecté en mesurant la fluorescence ; ou
ii) au moins une sonde s'hybridant spécifiquement au produit d'amplification et comprenant un marqueur détectable, de préférence ladite au moins une sonde étant un oligonucléotide constitué de SEQ ID 3.

6. Procédé selon l'une quelconque des revendications 1 à 5, dans lequel ladite étape de traitement i) comprend la lyse de cellules bactériennes, l'extraction et la purification d'acide nucléique, ce qui permet d'obtenir un acide nucléique bactérien purifié dans un tampon.

7. Procédé selon l'une quelconque des revendications 1 à 6, dans lequel ledit segment du gène d'ARNr 16S fait partie d'un ADNc obtenu par transcription inverse d'ARN au sein d'un ADNc à l'aide d'une transcriptase inverse.

8. Procédé selon l'une quelconque des revendications 1 à 7, dans lequel ledit segment du gène d'ARNr 16S comprend la séquence nucléotidique identifiée en tant que SEQ ID 4.

9. Procédé selon l'une quelconque des revendications 1 à 8, comprenant en outre la détection d'au moins un témoin négatif et/ou d'au moins un témoin positif.

10. Procédé selon la revendication 9, dans lequel ladite détection d'au moins un témoin négatif comprend l'amplification et la détection d'une séquence d'acide nucléique d'un plasmide ne contenant pas la séquence nucléotidique identifiée en tant que SEQ ID 4.

11. Procédé selon la revendication 9 ou 10, dans lequel ladite détection d'au moins un témoin positif comprend l'amplification et la détection d'une séquence d'acide nucléique d'un plasmide contenant la séquence nucléotidique identifiée en tant que SEQ ID 4.

12. Procédé selon l'une quelconque des revendications 1 à 11, dans lequel ledit échantillon est un fluide corporel de mammifère choisi dans le groupe constitué par le sang, les fractions sanguines, le plasma, la moelle osseuse, l'urine, les selles, la salive, la lymphe, les exsudats, les transsudats, les sécrétions, le liquide céphalorachidien, le liquide séminal, un tissu dispersé et/ou les fluides provenant de cavités corporelles naturelles ou non naturelles ou les frottis, ou un échantillon de culture de cellules.

13. Composition comprenant une paire d'amorces oligonucléotidiques constituée d'une amorce sens comprenant au moins 10 bases consécutives de SEQ ID 1 et d'une amorce antisens comprenant au moins 10 bases consécutives de SEQ ID 2.

14. Composition selon la revendication 13, dans laquelle l'amorce sens est constituée de SEQ ID 1 et l'amorce antisens est constituée de SEQ ID 2.

15. Composition selon la revendication 13 ou 14, qui est fournie dans un kit de pièces ou un réactif composite prêt à l'emploi dans une analyse par qPCR, de préférence dans laquelle la paire d'amorces est fournie dans un mélange.


### SEQUENCE LISTING

<110> Mycoplasma Biosafety Services GmbH
<120> HAEMOPLASMA DETECTION METHOD
<130> MY001EP
<160> 181
<170> PatentIn version 3.5
<210> 1
   <211> 23
   <212> DNA
   <213> Artificial Sequence
<220>
   <223> Primer
<400> 1
   agcaaatggg attagatacc cca 23
<210> 2
   <211> 25
   <212> DNA
   <213> Artificial Sequence
<220>
   <223> Primer
<400> 2
   ttcctttgag tttcatattt gcata 25
<210> 3
   <211> 24
   <212> DNA
   <213> Artificial Sequence
<220>
   <223> Probe
<400> 3
   catcgtttac ggtgtggact actg 24
<210> 4
   <211> 140
   <212> DNA
   <213> Artificial Sequence
<220>
   <223> Segment of the 16S rRNA gene
<220>
   <221> misc_feature
   <222> (2)..(2)
   <223> n at position 2 is any of G or T
<220>
   <221> misc_feature
   <222> (3)..(3)
   <223> n at position 3 is any of C or T
<220>
   <221> misc_feature
   <222> (33)..(33)
   <223> n at position 33 is any of A or G
<220>
   <221> misc_feature
   <222> (46)..(46)
   <223> n at position 46 is any of A or G
<220>
   <221> misc_feature
   <222> (48)..(48)
   <223> n at position 48 is any of G, C, A or T
<220>
   <221> misc_feature
   <222> (54)..(54)
   <223> n at position 54 is any of G, A or T
<220>
   <221> misc_feature
   <222> (55)..(55)
   <223> n at position 55 is any of C or T
<220>
   <221> misc_feature
   <222> (56)..(56)
   <223> n at position 56 is any of A or G
<220>
   <221> misc_feature
   <222> (58)..(58)
   <223> n at position 58 is any of G, T or C
<220>
   <221> misc_feature
   <222> (59)..(59)
   <223> n at position 59 is any of G, A or T
<220>
   <221> misc_feature
   <222> (61)..(61)
   <223> n at position 61 is any of A or G
<220>
   <221> misc_feature
   <222> (62)..(62)
   <223> n at position 62 is any of G, C, A or T
<220>
   <221> misc_feature
   <222> (63)..(63)
   <223> n at position 63 is any of G, C, A or T
<220>
   <221> misc_feature
   <222> (66)..(66)
   <223> n at position 66 is any of G, C, A or T
<220>
   <221> misc_feature
   <222> (67)..(67)
   <223> n at position 67 is any of G, C, A or T
<220>
   <221> misc_feature
   <222> (68)..(68)
   <223> n at position 68 is any of G, A or T
<220>
   <221> misc_feature
   <222> (69)..(69)
   <223> n at position 69 is any of G, C, A or T
<220>
   <221> misc_feature
   <222> (70)..(70)
   <223> n at position 70 is any of C or T
<220>
   <221> misc_feature
   <222> (71)..(71)
   <223> n at position 71 is any of C or T
<220>
   <221> misc_feature
   <222> (72)..(72)
   <223> n at position 72 is any of G, C, A or T
<220>
   <221> misc_feature
   <222> (73)..(73)
   <223> n at position 73 is any of A or G
<220>
   <221> misc_feature
   <222> (75)..(75)
   <223> n at position 75 is any of C or T
<220>
   <221> misc_feature
   <222> (85)..(85)
   <223> n at position 85 is any of A or T
<220>
   <221> misc_feature
   <222> (89)..(89)
   <223> n at position 89 is any of C or T
<220>
   <221> misc_feature
   <222> (97)..(97)
   <223> n at position 97 is any of G, A or T
<220>
   <221> misc_feature
   <222> (99)..(99)
   <223> n at position 99 is any of C or T
<220>
   <221> misc_feature
   <222> (114)..(114)
   <223> n at position 114 is any of C or T
<220>
   <221> misc_feature
   <222> (138)..(138)
   <223> n at position 138 is any of A or G
<400> 4
   annaaatggg attagatacc ccagtagtcc acnccgtaaa cgatgngnat tagnnntnng 60
   nnnttnnnnn nnngnghtgt agctnacgng ttaaatncnc cgcctgggta gtanatatgc 120
   aaatatgaaa ctcaaagnaa 140
<210> 5
   <211> 140
   <212> DNA
   <213> Haemobartonella felis
<400> 5
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 6
   <211> 140
   <212> DNA
   <213> Haemobartonella felis
<400> 6
   agcaaatggg attagatacc ccagtagtcc acaccgtaaa cgatgggtat tagatattag     60
   ggctttagct ttagtgttgt agcttacgcg ttaaataccc cgcctgggta gtacatatgc    120
   aaatatgaaa ctcaaaggaa                                                 140
<210> 7
   <211> 140
   <212> DNA
   <213> Haemobartonella felis
<400> 7
   agcaaatggg attagatacc ccagtagtcc acaccgtaaa cgatgggtat tagatattag     60
   ggctttagct ttagtgttgt agcttacgcg ttaaataccc cgcctgggta gtacatatgc    120
   aaatatgaaa ctcaaaggaa                                                 140
<210> 8
   <211> 140
   <212> DNA
   <213> Haemobartonella felis
<400> 8
   agcaaatggg attagatacc ccagtagtcc acaccgtaaa cgatgggtat tagatattag     60
   ggctttagct ttagtgttgt agcttacgcg ttaaataccc cgcctgggta gtacatatgc    120
   aaatatgaaa ctcaaaggaa                                                 140
<210> 9
   <211> 140
   <212> DNA
   <213> Haemobartonella felis
<400> 9
   agcaaatggg attagatacc ccagtagtcc acaccgtaaa cgatgggtat tagatattag     60
   ggctttagct ttagtgttgt agcttacgcg ttaaataccc cgcctgggta gtacatatgc    120
   aaatatgaaa ctcaaaggaa                                                 140
<210> 10
   <211> 140
   <212> DNA
   <213> Haemobartonella felis
<400> 10
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 11
   <211> 140
   <212> DNA
   <213> Haemobartonella felis
<400> 11
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 12
   <211> 140
   <212> DNA
   <213> Haemobartonella felis
<400> 12
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 13
   <211> 140
   <212> DNA
   <213> Haemobartonella felis
<400> 13
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 14
   <211> 140
   <212> DNA
   <213> Haemobartonella felis
<400> 14
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 15
   <211> 140
   <212> DNA
   <213> Haemobartonella felis
<400> 15
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 16
   <211> 140
   <212> DNA
   <213> Haemobartonella felis
<400> 16
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 17
   <211> 140
   <212> DNA
   <213> Haemobartonella felis
<400> 17
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 18
   <211> 140
   <212> DNA
   <213> Haemobartonella felis
<400> 18
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 19
   <211> 140
   <212> DNA
   <213> Haemobartonella felis
<400> 19
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 20
   <211> 140
   <212> DNA
   <213> Artificial Sequence
<220>
   <223> Uncultured Mycoplasma sp. clone Mh.1-BR
<400> 20
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 21
   <211> 140
   <212> DNA
   <213> Haemobartonella canis
<400> 21
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 22
   <211> 140
   <212> DNA
   <213> Haemobartonella felis
<400> 22
   agcaaatggg attagatacc ccagtagtcc acgccgtaaa cgatgggtat tagatattag 60
   ggctttagct ttagtgttga tgcttacgcg ttaaataccc cgcctgggta gtacatatgc 120
   aaatatgaaa ctcaaaggaa 140
<210> 23
   <211> 140
   <212> DNA
   <213> Haemobartonella felis
<400> 23
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 24
   <211> 140
   <212> DNA
   <213> Haemobartonella felis
<400> 24
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 25
   <211> 140
   <212> DNA
   <213> Haemobartonella felis
<400> 25
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 26
   <211> 140
   <212> DNA
   <213> Haemobartonella felis
<400> 26
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 27
   <211> 140
   <212> DNA
   <213> Haemobartonella felis
<400> 27
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 28
   <211> 140
   <212> DNA
   <213> Haemobartonella felis
<400> 28
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 29
   <211> 140
   <212> DNA
   <213> Haemobartonella felis
<400> 29
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 30
   <211> 140
   <212> DNA
   <213> Haemobartonella felis
<400> 30
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 31
   <211> 140
   <212> DNA
   <213> Haemobartonella felis
<400> 31
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 32
   <211> 140
   <212> DNA
   <213> Haemobartonella felis
<400> 32
   agcaaatggg attagatacc ccagtagtcc acaccgtaaa cgatgggtat tagatattag        60
   ggctttagct ttagtgttgt agcttacgcg ttaaataccc cgcctgggta gtacatatgc       120
   aaatatgaaa ctcaaaggaa                                                   140
<210> 33
   <211> 140
   <212> DNA
   <213> Haemobartonella felis
<400> 33
   agcaaatggg attagatacc ccagtagtcc acaccgtaaa cgatgggtat tagatattag        60
   ggctttagct ttagtgttgt agcttacgcg ttaaataccc cgcctgggta gtacatatgc       120
   aaatatgaaa ctcaaaggaa                                                   140
<210> 34
   <211> 140
   <212> DNA
   <213> Haemobartonella felis
<400> 34
   agcaaatggg attagatacc ccagtagtcc acaccgtaaa cgatgggtat tagatattag        60
   ggctttagct ttagtgttgt agcttacgcg ttaaataccc cgcctgggta gtacatatgc       120
   aaatatgaaa ctcaaaggaa                                                   140
<210> 35
   <211> 140
   <212> DNA
   <213> Haemobartonella felis
<400> 35
   agcaaatggg attagatacc ccagtagtcc acaccgtaaa cgatgggtat tagatattag        60
   ggctttagct ttagtgttgt agcttacgcg ttaaataccc cgcctgggta gtacatatgc       120
   aaatatgaaa ctcaaaggaa                                                   140
<210> 36
   <211> 140
   <212> DNA
   <213> Haemobartonella felis
<400> 36
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 37
   <211> 140
   <212> DNA
   <213> Haemobartonella felis
<400> 37
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 38
   <211> 140
   <212> DNA
   <213> Haemobartonella felis
<400> 38
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 39
   <211> 140
   <212> DNA
   <213> Haemobartonella felis
<400> 39
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 40
   <211> 140
   <212> DNA
   <213> Haemobartonella felis
<400> 40
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 41
   <211> 140
   <212> DNA
   <213> Haemobartonella felis
<400> 41
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 42
   <211> 140
   <212> DNA
   <213> Haemobartonella felis
<400> 42
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 43
   <211> 140
   <212> DNA
   <213> Haemobartonella felis
<400> 43
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 44
   <211> 140
   <212> DNA
   <213> Haemobartonella felis
<400> 44
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 45
   <211> 140
   <212> DNA
   <213> Haemobartonella felis
<400> 45
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 46
   <211> 140
   <212> DNA
   <213> Haemobartonella felis
<400> 46
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 47
   <211> 140
   <212> DNA
   <213> Haemobartonella canis
<400> 47
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 48
   <211> 140
   <212> DNA
   <213> Haemobartonella canis
<400> 48
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 49
   <211> 140
   <212> DNA
   <213> Haemobartonella canis
<400> 49
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 50
   <211> 140
   <212> DNA
   <213> Haemobartonella canis
<400> 50
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 51
   <211> 140
   <212> DNA
   <213> Haemobartonella canis
<400> 51
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 52
   <211> 140
   <212> DNA
   <213> Haemobartonella canis
<400> 52
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 53
   <211> 140
   <212> DNA
   <213> Haemobartonella canis
<400> 53
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 54
   <211> 140
   <212> DNA
   <213> Haemobartonella canis
<400> 54
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 55
   <211> 140
   <212> DNA
   <213> Haemobartonella canis
<400> 55
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 56
   <211> 140
   <212> DNA
   <213> Haemobartonella canis
<400> 56
   agcaaatggg attagatacc ccagtagtcc acaccgtaaa cgatgggtat tagatattag 60
   ggctttagct ttagtgttgt agcttacgcg ttaaataccc cgcctgggta gtacatatgc 120
   aaatatgaaa ctcaaaggaa 140
<210> 57
   <211> 140
   <212> DNA
   <213> Haemobartonella muris
<400> 57
   agcaaatggg attagatacc ccagtagtcc acaccgtaaa cgatggatat tagatgttgg 60
   gacttgagtc tcagcgttgt agcttacgtg ttaaatatcc cgcctgagta gtacatatgc 120
   aaatatgaaa ctcaaaggaa 140
<210> 58
   <211> 140
   <212> DNA
   <213> Artificial sequence
<220>
   <223> Candidatus Mycoplasma haemohominis clone Plymouth
<400> 58
   agcaaatggg attagatacc ccagtagtcc acaccgtaaa cgatggatat tagatgttgg 60
   gacttgcgtc tcagcgttgt agcttacgtg ttaaatatcc cgcctgggta gtacatatgc 120
   aaatatgaaa ctcaaaggaa 140
<210> 59
   <211> 140
   <212> DNA
   <213> Artificial sequence
<220>
   <223> Mycoplasma sp. 'feline hemotropic Switzerland'
<400> 59
   agcaaatggg attagatacc ccagtagtcc acaccgtaaa cgatgggtat tagatgtcgg 60
   gatttgtgtt tcggcgttgt agcttacgtg ttaaataccc cgcctgggta gtacatatgc 120
   aaatatgaaa ctcaaaggaa 140
<210> 60
   <211> 140
   <212> DNA
   <213> Artificial sequence
<220>
   <223> Candidatus Mycoplasma turicensis strain USP-14
<400> 60
   agcaaatggg attagatacc ccagtagtcc acaccgtaaa cgatgggtat tagatgtcgg 60
   gacttgagtt tcggcgttgt agcttacgtg ttaaataccc cgcctgggta gtacatatgc 120
   aaatatgaaa ctcaaaggaa 140
<210> 61
   <211> 140
   <212> DNA
   <213> Artificial sequence
<220>
   <223> Candidatus Mycoplasma turicensis clone 2.24
<400> 61
   agcaaatggg attagatacc ccagtagtcc acaccgtaaa cgatgggtat tagatgtcgg 60
   gatttgtgtt tcggcgttgt agcttacgtg ttaaataccc cgcctgggta gtacatatgc 120
   aaatatgaaa ctcaaaggag 140
<210> 62
   <211> 141
   <212> DNA
   <213> Artificial sequence
<220>
   <223> Candidatus Mycoplasma turicensis isolate D1
<400> 62
   agcaaatggg attagatacc ccagtagtcc acaccgtaaa cgatgggtat taggatgtcg 60
   ggatttgtgt ttcggcgttg tagcttacgt gttaaatacc ccgcctgggt agtacatatg 120
   caaatatgaa actcaaagga a 141
<210> 63
   <211> 140
   <212> DNA
   <213> Artificial sequence
<220>
   <223> Candidatus Mycoplasma turicensis isolate 108N
<400> 63
   agcaaatggg attagatacc ccagtagtcc acaccgtaaa cgatgggtat tagatgtcgg 60
   gatttgtgtt tcggcgttgt agcttacgtg ttaaataccc cgcctgggta gtacatatgc 120
   aaatatgaaa ctcaaaggaa 140
<210> 64
   <211> 140
   <212> DNA
   <213> Artificial sequence
<220>
   <223> Candidatus Mycoplasma turicensis isolate D7
<400> 64
   agcaaatggg attagatacc ccagtagtcc acaccgtaaa cgatgggtat tagatgtcgg 60
   gacttgagtt tcggcgttgt agcttacgtg ttaaataccc cgcctgggta gtacatatgc 120
   aaatatgaaa ctcaaaggaa 140
<210> 65
   <211> 140
   <212> DNA
   <213> Artificial sequence
<220>
   <223> Candidatus Mycoplasma turicensis isolate D9
<400> 65
   agcaaatggg attagatacc ccagtagtcc acaccgtaaa cgatgggtat tagatgtcgg 60
   gacttgagtt tcggcgttgt agcttacgtg ttaaataccc cgcctgggta gtacatatgc 120
   aaatatgaaa ctcaaaggaa 140
<210> 66
   <211> 140
   <212> DNA
   <213> Artificial sequence
<220>
   <223> Candidatus Mycoplasma turicensis isolate 22
<400> 66
   agcaaatggg attagatacc ccagtagtcc acaccgtaaa cgatgggtat tagatgtcgg      60
   gatttgtgtt tcggcgttgt agcttacgtg ttaaataccc cgcctgggta gtacatatgc     120
   aaatatgaaa ctcaaaggaa                                                 140
<210> 67
   <211> 140
   <212> DNA
   <213> Artificial sequence
<220>
   <223> Candidatus Mycoplasma turicensis isolate 4
<400> 67
   agcaaatggg attagatacc ccagtagtcc acaccgtaaa cgatgggtat tagatgtcgg      60
   gatttgtgtt tcggcgttgt agcttacgtg ttaaataccc cgcctgggta gtacatatgc     120
   aaatatgaaa ctcaaaggaa                                                 140
<210> 68
   <211> 140
   <212> DNA
   <213> Artificial sequence
<220>
   <223> Candidatus Mycoplasma turicensis isolate 10
<400> 68
   agcaaatggg attagatacc ccagtagtcc acaccgtaaa cgatgggtat tagatgtcgg      60
   gatttgtgtt tcggcgttgt agcttacgtg ttaaataccc cgcctgggta gtacatatgc     120
   aaatatgaaa ctcaaaggaa                                                 140
<210> 69
   <211> 140
   <212> DNA
   <213> Artificial sequence
<220>
   <223> Candidatus Mycoplasma turicensis isolate 94-100
<400> 69
   agcaaatggg attagatacc ccagtagtcc acaccgtaaa cgatgggtat tagatgtcgg 60
   gatttgaatt tcggcgttgt agcttacgtg ttaaataccc cgcctgggta gtacatatgc 120
   aaatatgaaa ctcaaaggaa 140
<210> 70
   <211> 140
   <212> DNA
   <213> Artificial sequence
<220>
   <223> Candidatus Mycoplasma turicensis isolate Porto Alegre
<400> 70
   agcaaatggg attagatacc ccagtagtcc acaccgtaaa cgatgggtat tagatgtcgg 60
   gatttgtgtt tcggcgttgt agcttacgtg ttaaataccc cgcctgggta gtacatatgc 120
   aaatatgaaa ctcaaaggaa 140
<210> 71
   <211> 140
   <212> DNA
   <213> Artificial sequence
<220>
   <223> Mycoplasma coccoides
<400> 71
   agcaaatggg attagatacc ccagtagtcc acaccgtaaa cgatgggtat taggtgccgg 60
   ggttagagct tcggtgctgt agcttacgtg ttaaataccc cgcctgggta gtacatatgc 120
   aaatatgaaa ctcaaaggaa 140
<210> 72
   <211> 140
   <212> DNA
   <213> Artificial sequence
<220>
   <223> Uncultured Mycoplasma sp. clone 1
<400> 72
   agcaaatggg attagatacc ccagtagtcc acaccgtaaa cgatgggtat tggatgtcgg 60
   gctttgtggc tcggtgttgt agcttacgtg ttaaataccc cgcctgggta gtatatatgc 120
   aaatatgaaa ctcaaaggaa 140
<210> 73
   <211> 140
   <212> DNA
   <213> Artificial sequence
<220>
   <223> Uncultured Mycoplasma sp. clone 2
<400> 73
   agcaaatggg attagatacc ccagtagtcc acaccgtaaa cgatgggtat tggatgtcgg      60
   gctttgcggc tcggtgttgt agcttacgtg ttaaataccc cgcctgggta gtatatatgc     120
   aaatatgaaa ctcaaaggaa                                                 140
<210> 74
   <211> 140
   <212> DNA
   <213> Artificial sequence
<220>
   <223> Candidatus Mycoplasma haemomacaque
<400> 74
   agcaaatggg attagatacc ccagtagtcc acaccgtaaa cgatggatat tagatgttgg      60
   ggtctgtgcc tcagcgttgt agcttacgtg ttaaatatcc cgcctgggta gtacatatgc     120
   aaatatgaaa ctcaaaggaa                                                 140
<210> 75
   <211> 141
   <212> DNA
   <213> Artificial sequence
<220>
   <223> Candidatus Mycoplasma haemobos
<400> 75
   agcaagtggg attagatacc ccagtagttc cacaccgtaa acgatgggta ttaggtattg      60
   gggtttgagc ctcagtgctg tagctaacgt gttaaatacc ccgcctgggt agtacatatg     120
   caaataagaa actcaaagga a                                               141
<210> 76
   <211> 140
   <212> DNA
   <213> Artificial sequence
<220>
   <223> Candidatus Mycoplasma haemobos clone 311
<400> 76
   agcaaatggg attagatacc ccagtagtcc acaccgtaaa cgatgggtat taggtattgg 60
   ggtttgagcc tcagtgctgt agctaacgtg ttaaataccc cgcctgggta gtacatatgc 120
   aaatatgaaa ctcaaaggaa 140
<210> 77
   <211> 139
   <212> DNA
   <213> Artificial sequence
<220>
   <223> Candidatus Mycoplasma haemovis
<400> 77
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 78
   <211> 139
   <212> DNA
   <213> Artificial sequence
<220>
   <223> Candidatus Mycoplasma haemovis
<400> 78
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 79
   <211> 139
   <212> DNA
   <213> Artificial sequence
<220>
   <223> Candidatus Mycoplasma haemovis
<400> 79
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 80
   <211> 139
   <212> DNA
   <213> Artificial sequence
<220>
   <223> Candidatus Mycoplasma haemovis
<400> 80
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 81
   <211> 139
   <212> DNA
   <213> Artificial sequence
<220>
   <223> Candidatus Mycoplasma haemovis
<400> 81
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 82
   <211> 139
   <212> DNA
   <213> Artificial sequence
<220>
   <223> Candidatus Mycoplasma haemovis isolate CMho-Hu-1-05.2007
<400> 82
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 83
   <211> 139
   <212> DNA
   <213> Artificial sequence
<220>
   <223> Candidatus Mycoplasma haemovis isolate CMho-Hu-2-05.2007
<400> 83
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 84
   <211> 139
   <212> DNA
   <213> Artificial sequence
<220>
   <223> Candidatus Mycoplasma haemovis isolate CMho-Hu-3-05.2007
<400> 84
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 85
   <211> 139
   <212> DNA
   <213> Artificial sequence
<220>
   <223> Candidatus Mycoplasma haemovis isolate CMho-Hu-4.05-2007
<400> 85
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 86
   <211> 139
   <212> DNA
   <213> Artificial sequence
<220>
   <223> Candidatus Mycoplasma haemovis isolate CMho-Hu-5-05.2007
<400> 86
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 87
   <211> 139
   <212> DNA
   <213> Artificial sequence
<220>
   <223> Candidatus Mycoplasma haemovis isolate TA1
<400> 87
   agtaaatggg attagatacc ccagtagtca acgccgtaaa cgatgagcat taggtatttg      60
   atgttaggtc gagtgctgta gctaacgcgt taaatgctcc gcctgggtag tatatatgca     120
   aatatgaaac tcaacgaaa                                                  139
<210> 88
   <211> 139
   <212> DNA
   <213> Artificial sequence
<220>
   <223> Candidatus Mycoplasma haemovis isolate TA2
<400> 88
   agtaaatggg attagatacc ccagtagtca acgccgtaaa cgatgagcat taggtatttg      60
   atgttaggtc gagtgctgta gctaacgcgt taaatgctcc gcctgggtag tatatatgca     120
   aatatgaaac tcaacgaaa                                                  139
<210> 89
   <211> 139
   <212> DNA
   <213> Artificial sequence
<220>
   <223> Candidatus Mycoplasma haemovis isolate TA3
<400> 89
   agtaaatggg attagatacc ccagtagtca acgccgtaaa cgatgagcat taggtatttg      60
   atgttaggtc gagtgctgta gctaacgcgt taaatgctcc gcctgggtag tatatatgca     120
   aatatgaaac tcaacgaaa                                                  139
<210> 90
   <211> 139
   <212> DNA
   <213> Artificial sequence
<220>
   <223> Candidatus Mycoplasma haemovis isolate TA6
<400> 90
   agtaaatggg attagatacc ccagtagtca acgccgtaaa cgatgagcat taggtatttg 60
   atgttaggtc gagtgctgta gctaacgcgt taaatgctcc gcctgggtag tatatatgca 120
   aatatgaaac tcaacgaaa 139
<210> 91
   <211> 139
   <212> DNA
   <213> Artificial sequence
<220>
   <223> Candidatus Mycoplasma haemovis isolate TA7
<400> 91
   agtaaatggg attagatacc ccagtagtca acgccgtaaa cgatgagcat taggtatttg 60
   atgttaggtc gagtgctgta gctaacgcgt tagatgctcc gcctgggtag tatatatgca 120
   aatatgaaac tcaacgaaa 139
<210> 92
   <211> 139
   <212> DNA
   <213> Artificial sequence
<220>
   <223> Mycoplasma ovis
<400> 92
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 93
   <211> 139
   <212> DNA
   <213> Artificial sequence
<220>
   <223> Mycoplasma ovis str. Michigan
<400> 93
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 94
   <211> 139
   <212> DNA
   <213> Artificial sequence
<220>
   <223> Mycoplasma ovis str. Michigan
<400> 94
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 95
   <211> 139
   <212> DNA
   <213> Artificial sequence
<220>
   <223> Mycoplasma ovis isolate 1294
<400> 95
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 96
   <211> 139
   <212> DNA
   <213> Artificial sequence
<220>
   <223> Mycoplasma ovis isolate Mo-Hu-1-05.2007
<400> 96
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 97
   <211> 139
   <212> DNA
   <213> Artificial sequence
<220>
   <223> Mycoplasma ovis isolate Mo-Hu-2-05.2007
<400> 97
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 98
   <211> 139
   <212> DNA
   <213> Artificial sequence
<220>
   <223> Mycoplasma ovis isolate Mo-Hu-3-05.2007
<400> 98
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 99
   <211> 139
   <212> DNA
   <213> Artificial sequence
<220>
   <223> Mycoplasma ovis isolate Mo-Hu-4-05.2007
<400> 99
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 100
   <211> 139
   <212> DNA
   <213> Artificial sequence
<220>
   <223> Mycoplasma ovis isolate TA4
<400> 100
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaacgaaa 139
<210> 101
   <211> 139
   <212> DNA
   <213> Artificial sequence
<220>
   <223> Mycoplasma ovis isolate TA5
<400> 101
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 102
   <211> 139
   <212> DNA
   <213> Artificial sequence
<220>
   <223> Mycoplasma ovis isolate TA9
<400> 102
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 103
   <211> 139
   <212> DNA
   <213> Artificial sequence
<220>
   <223> Mycoplasma ovis strain hangzhoul
<400> 103
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 104
   <211> 139
   <212> DNA
   <213> Artificial sequence
<220>
   <223> Mycoplasma ovis strain Shihezil
<400> 104
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 105
   <211> 139
   <212> DNA
   <213> Artificial sequence
<220>
   <223> Mycoplasma ovis strain TX1294-B
<400> 105
   agtaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 106
   <211> 139
   <212> DNA
   <213> Artificial sequence
<220>
   <223> Mycoplasma ovis strain TX1294-C
<400> 106
   agcaaatggg attagatacc tcagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 107
   <211> 139
   <212> DNA
   <213> Artificial sequence
<220>
   <223> Mycoplasma ovis strain TX1294-D
<400> 107
   agcaaatggg attagatacc ccagtagtcc acgccgtaaa cgatgagcat taggtatttg 60
   atgttaggtc gagtgctgta gctaacgcgt taaatgctcc gcctgggtag tatatatgca 120
   aatatgaaac tcaaagaaa 139
<210> 108
   <211> 139
   <212> DNA
   <213> Artificial sequence
<220>
   <223> Uncultured Mycoplasma sp. clone 0221
<400> 108
   agcaaatggg attagatacc ccagtagtcc acgccgtaaa cgatgagtat taggtatttg 60
   atgttatgtc gagtgctgta gctaacgcgt taaatactcc gcctgggtag tatatatgca 120
   aatatgaaac tcaaagaaa 139
<210> 109
   <211> 139
   <212> DNA
   <213> Artificial sequence
<220>
   <223> Uncultured Mycoplasma sp. clone 1437
<400> 109
   agcaaatggg attagatacc ccagtagtcc acgccgtaaa cgatgagaat taggtatttg 60
   atgttatatc gagtgctgta gctaacgcgt taaattctcc gcctgggtag tatatatgca 120
   aatatgaaac tcaaagaaa 139
<210> 110
   <211> 139
   <212> DNA
   <213> Artificial sequence
<220>
   <223> Uncultured Mycoplasma sp. clone 1585
<400> 110
   agcaaatggg attagatacc ccagtagtcc acgccgtaaa cgatgggtat taggtatttg 60
   atttaatatt gagtgctgta gctaacgcgt taaatacccc gcctgggtag tatatatgca 120
   aatatgaaac tcaaagaaa 139
<210> 111
   <211> 139
   <212> DNA
   <213> Artificial sequence
<220>
   <223> Uncultured Mycoplasma sp. clone 2167
<400> 111
   agcaaatggg attagatacc ccagtagtcc acgccgtaaa cgatgagaat taggtatttg 60
   atgttatatc gagtgctgta gctaacgcgt taaattctcc gcctgggtag tatatatgca 120
   aatatgaaac tcaaagaaa 139
<210> 112
   <211> 139
   <212> DNA
   <213> Artificial sequence
<220>
   <223> Uncultured Mycoplasma sp. clone 2189
<400> 112
   agcaaatggg attagatacc ccagtagtcc acgccgtaaa cgatgagaat taggtatttg 60
   atgttatatc gagtgctgta gctaacgcgt taaattctcc gcctgggtag tatatatgca 120
   aatatgaaac tcaaagaaa 139
<210> 113
   <211> 139
   <212> DNA
   <213> Artificial sequence
<220>
   <223> Uncultured Mycoplasma sp. clone 2238
<400> 113
   agcaaatggg attagatacc ccagtagtcc acgccgtaaa cgatgagaat taggtatttg 60
   atgttatatc gagtgctgta gctaacgcgt taaattctcc gcctgggtag tatatatgca 120
   aatatgaaac tcaaagaaa 139
<210> 114
   <211> 139
   <212> DNA
   <213> Artificial sequence
<220>
   <223> Uncultured Mycoplasma sp. clone 2385
<400> 114
   agcaaatggg attagatacc ccagtagtcc acgccgtaaa cgatgagaat taggtatttg 60
   atgttatatc gagtgctgta gctaacgcgt taaattctcc gcctgggtag tatatatgca 120
   aatatgaaac tcaaagaaa 139
<210> 115
   <211> 139
   <212> DNA
   <213> Artificial sequence
<220>
   <223> Uncultured Mycoplasma sp. clone 2404
<400> 115
   agcaaatggg attagatacc ccagtagtcc acgccgtaaa cgatgagaat taggtatttg 60
   atgttatatc gagtgctgta gctaacgcgt taaattctcc gcctgggtag tatatatgca 120
   aatatgaaac tcaaagaaa 139
<210> 116
   <211> 140
   <212> DNA
   <213> Artificial sequence
<220>
   <223> Mycoplasma parvum gene
<400> 116
   agcaaatggg attagatacc ccagtagtcc acgccgtaaa cgatgggtat tagtcatttg 60
   gatttaagac tgagtgatgt agctaacgcg ttaaataccc cgcctgggta gtatatatgc 120
   aaatatgaaa ctcaaagaaa 140
<210> 117
   <211> 140
   <212> DNA
   <213> Artificial sequence
<220>
   <223> Mycoplasma parvum gene
<400> 117
   agcaaatggg attagatacc ccagtagtcc acgccgtaaa cgatgggtat tagtcatttg 60
   gatttaagac tgagtgatgt agctaacgcg ttaaataccc cgcctgggta gtatatatgc 120
   aaatatgaaa ctcaaagaaa 140
<210> 118
   <211> 140
   <212> DNA
   <213> Artificial sequence
<220>
   <223> Mycoplasma parvum gene
<400> 118
   agcaaatggg attagatacc ccagtagtcc acgccgtaaa cgatgggtat tagtcatttg 60
   gatttaagac tgagtgatgt agctaacgcg ttaaataccc cgcctgggta gtatatatgc 120
   aaatatgaaa ctcaaagaaa 140
<210> 119
   <211> 140
   <212> DNA
   <213> Artificial sequence
<220>
   <223> Mycoplasma parvum str. Indiana
<400> 119
   agcaaatggg attagatacc ccagtagtcc acgccgtaaa cgatgggtat tagtcatttg 60
   gatttaagac tgagtgatgt agctaacgcg ttaaataccc cgcctgggta gtatatatgc 120
   aaatatgaaa ctcaaagaaa 140
<210> 120
   <211> 139
   <212> DNA
   <213> Artificial sequence
<220>
   <223> Candidatus Mycoplasma haemominutum
<400> 120
   agcaaatggg attagatacc ccagtagtcc acgccgtaaa cgatgggtat taggtatttg       60
   gtctaggact gagtgctgta gctaacgcgt taaatacccc gcctgggtag tatatatgca      120
   aatatgaaac tcaaagaaa                                                   139
<210> 121
   <211> 139
   <212> DNA
   <213> Artificial sequence
<220>
   <223> Candidatus Mycoplasma haemominutum isolate Israel no. 1
<400> 121
   agcaaatggg attagatacc ccagtagtcc acgccgtaaa cgatgggtat taggtatttg       60
   gtttaggact gagtgctgta gctaacgcgt taaatacccc gcctgggtag tatatatgca      120
   aatatgaaac tcaaagaaa                                                   139
<210> 122
   <211> 139
   <212> DNA
   <213> Artificial sequence
<220>
   <223> Candidatus Mycoplasma haemominutum isolate Australian no. 3
<400> 122
   agcaaatggg attagatacc ccagtagtcc acgccgtaaa cgatgggtat taggtatttg       60
   gtctaggact gagtgctgta gctaacgcgt taaatacccc gcctgggtag tatatatgca      120
   aatatgaaac tcaaagaaa                                                   139
<210> 123
   <211> 139
   <212> DNA
   <213> Artificial sequence
<220>
   <223> Candidatus Mycoplasma haemominutum isolate South African no. 1
<400> 123
   agcaaatggg attagatacc ccagtagtcc acgccgtaaa cgatgggtat taggtatttg 60
   gtctaggact gagtgctgta gctaacgcgt taaatacccc gcctgggtag tatatatgca 120
   aatatgaaac tcaaagaaa 139
<210> 124
   <211> 139
   <212> DNA
   <213> Artificial sequence
<220>
   <223> Candidatus Mycoplasma haemominutum isolate UK no. 1
<400> 124
   agcaaatggg attagatacc ccagtagtcc acgccgtaaa cgatgggtat taggtatttg 60
   gtctaggact gagtgctgta gctaacgcgt taaatacccc gcctgggtag tatatatgca 120
   aatatgaaac tcaaagaaa 139
<210> 125
   <211> 139
   <212> DNA
   <213> Artificial sequence
<220>
   <223> Candidatus Mycoplasma haemominutum isolate UK no. 2
<400> 125
   agcaaatggg attagatacc ccagtagtcc acgccgtaaa cgatgggtat taggtatttg 60
   gtctaggact gagtgctgta gctaacgcgt taaatacccc gcctgggtag tatatatgca 120
   aatatgaaac tcaaagaaa 139
<210> 126
   <211> 139
   <212> DNA
   <213> Artificial sequence
<220>
   <223> Candidatus Mycoplasma haemominutum
<400> 126
   agcaaatggg attagatacc ccagtagtcc acgccgtaaa cgatgggtat taggtatttg 60
   gttaaggact gagtgctgta gctaacgcgt taaatacccc gcctgggtag tatatatgca 120
   aatatgaaac tcaaagaaa 139
<210> 127
   <211> 139
   <212> DNA
   <213> Artificial sequence
<220>
   <223> Candidatus Mycoplasma haemominutum
<400> 127
   agcaaatggg attagatacc ccagtagtcc acgccgtaaa cgatgggtat taggtatttg      60
   gtctaggact gagtgctgta gctaacgcgt taaatacccc gcctgggtag tatatatgca     120
   aatatgaaac tcaaagaaa                                                  139
<210> 128
   <211> 139
   <212> DNA
   <213> Artificial sequence
<220>
   <223> Candidatus Mycoplasma haemominutum clone 402799.14
<400> 128
   agcaaatggg attagatacc ccagtagtcc acgccgtaaa cgatgggtat taggtatttg      60
   gtctaggact gagtgctgta gctaacgcgt taaatacccc gcctgggtag tatatatgca     120
   aatatgaaac tcaaagaaa                                                  139
<210> 129
   <211> 139
   <212> DNA
   <213> Artificial sequence
<220>
   <223> Candidatus Mycoplasma haemominutum isolate 68
<400> 129
   agcaaatggg attagatacc ccagtagtcc acgccgtaaa cgatgggtat taggtatttg      60
   gtttaggact gagtgctgta gctaacgcgt taaatacccc gcctgggtag tatatatgca     120
   aatatgaaac tcaaagaaa                                                  139
<210> 130
   <211> 139
   <212> DNA
   <213> Artificial sequence
<220>
   <223> Candidatus Mycoplasma haemominutum isolate 111
<400> 130
   agcaaatggg attagatacc ccagtagtcc acgccgtaaa cgatgggtat taggtatttg      60
   gtttaggact gagtgctgta gctaacgcgt taaatacccc gcctgggtag tatatatgca     120
   aatatgaaac tcaaagaaa                                                  139
<210> 131
   <211> 139
   <212> DNA
   <213> Artificial sequence
<220>
   <223> Candidatus Mycoplasma haemominutum isolate 16
<400> 131
   agcaaatggg attagatacc ccagtagtcc acgccgtaaa cgatgggtat taggtatttg 60
   gtctaggact gagtgctgta gctaacgcgt taaatacccc gcctgggtag tatatatgca 120
   aatatgaaac tcaaagaaa 139
<210> 132
   <211> 139
   <212> DNA
   <213> Artificial sequence
<220>
   <223> Candidatus Mycoplasma haemominutum isolate 18
<400> 132
   agcaaatggg attagatacc ccagtagtcc acgccgtaaa cgatgggtat taggtatctg 60
   gtctaggact gagtgctgta gctaacgcgt taaatacccc gcctgggtag tatatatgca 120
   aatatgaaac tcaaagaaa 139
<210> 133
   <211> 139
   <212> DNA
   <213> Artificial sequence
<220>
   <223> Candidatus Mycoplasma haemominutum isolate 187
<400> 133
   agcaaatggg attagatacc ccagtagtcc acgccgtaaa cgatgggtat taggtatttg 60
   gtttaggact gagtgctgta gctaacgcgt taaatacccc gcctgggtag tatatatgca 120
   aatatgaaac tcaaagaaa 139
<210> 134
   <211> 139
   <212> DNA
   <213> Artificial sequence
<220>
   <223> Candidatus Mycoplasma haemominutum isolate 192
<400> 134
   agcaaatggg attagatacc ccagtagtcc acgccgtaaa cgatgggtat taggtatttg 60
   gtttaggact gagtgctgta gctaacgcgt taaatacccc gcctgggtag tatatatgca 120
   aatatgaaac tcaaagaaa 139
<210> 135
   <211> 139
   <212> DNA
   <213> Artificial sequence
<220>
   <223> Candidatus Mycoplasma haemominutum isolate 209
<400> 135
   agcaaatggg attagatacc ccagtagtcc acgccgtaaa cgatgggtat taggtatttg 60
   gtttaggact gagtgctgta gctaacgcgt taaatacccc gcctgggtag tatatatgca 120
   aatatgaaac tcaaagaaa 139
<210> 136
   <211> 139
   <212> DNA
   <213> Artificial sequence
<220>
   <223> Candidatus Mycoplasma haemominutum isolate 94-111
<400> 136
   agcaaatggg attagatacc ccagtagtcc acgccgtaaa cgatgggtat taggtatttg 60
   gtttaggact gagtgctgta gctaacgcgt taaatacccc gcctgggtag tatatatgca 120
   aatatgaaac tcaaagaaa 139
<210> 137
   <211> 139
   <212> DNA
   <213> Artificial sequence
<220>
   <223> Candidatus Mycoplasma haemominutum isolate 94-91
<400> 137
   agcaaatggg attagatacc ccagtagtcc acgccgtaaa cgatgggtat taggtatttg 60
   gtttaggact gagtgctgta gctaacgcgt taaatacccc gcctgggtag tatatatgca 120
   aatatgaaac tcaaagaaa 139
<210> 138
   <211> 139
   <212> DNA
   <213> Artificial sequence
<220>
   <223> Candidatus Mycoplasma haemominutum isolate 5235
<400> 138
   agcaaatggg attagatacc ccagtagtcc acgccgtaaa cgatgggtat taggtatttg      60
   gtttaggact gagtgctgta gctaacgcgt taaatacccc gcctgggtag tatatatgca     120
   aatatgaaac tcaaagaaa                                                  139
<210> 139
   <211> 139
   <212> DNA
   <213> Artificial sequence
<220>
   <223> Candidatus Mycoplasma haemominutum isolate Z7
<400> 139
   agcaaatggg attagatacc ccagtagtcc acgccgtaaa cgatgggtat taggtatttg      60
   gtttaggact gagtgctgta gctaacgcgt taaatacccc gcctgggtag tatatatgca     120
   aatatgaaac tcaaagaaa                                                  139
<210> 140
   <211> 139
   <212> DNA
   <213> Artificial sequence
<220>
   <223> Candidatus Mycoplasma haemominutum isolate Purdue
<400> 140
   agcaaatggg attagatacc ccagtagtcc acgccgtaaa cgatgggtat taggtatttg      60
   gtttaggact gagtgctgta gctaacgcgt taaatacccc gcctgggtag tatatatgca     120
   aatatgaaac tcaaagaaa                                                  139
<210> 141
   <211> 139
   <212> DNA
   <213> Artificial sequence
<220>
   <223> Candidatus Mycoplasma haemominutum strain California
<400> 141
   agcaaatggg attagatacc ccagtagtcc acgccgtaaa cgatgggtat taggtatttg 60
   gtctaggact gagtgctgta gctaacgcgt taaatacccc gcctgggtag tatatatgca 120
   aatatgaaac tcaaagaaa 139
<210> 142
   <211> 139
   <212> DNA
   <213> Artificial sequence
<220>
   <223> Candidatus Mycoplasma haemoparvum
<400> 142
   agcaaatggg attagatacc ccagtagtcc acgccgtaaa cgatggggat taggtatgtg 60
   atataagatt gagtgctgta gctaacgcgt taaatacccc gcctgggtag tatatatgca 120
   aatatgaaac tcaaagaaa 139
<210> 143
   <211> 139
   <212> DNA
   <213> Artificial sequence
<220>
   <223> Candidatus Mycoplasma haematoparvum clone 8591
<400> 143
   agcaaatggg attagatacc ccagtagtcc acgccgtaaa cgatgggtat taggtatttg 60
   atataagatt gagtgctgta gctaacgcgt taaatacccc gcctgggtag tatatatgca 120
   aatatgaaac tcaaagaaa 139
<210> 144
   <211> 139
   <212> DNA
   <213> Artificial sequence
<220>
   <223> Candidatus Mycoplasma haematoparvum clone 19686
<400> 144
   agcaaatggg attagatacc ccagtagtcc acgccgtaaa cgatgggtat taggtatttg 60
   atataagatt gagtgctgta gctaacgcgt taaatacccc gcctgggtag tatatatgca 120
   aatatgaaac tcaaagaaa 139
<210> 145
   <211> 139
   <212> DNA
   <213> Artificial sequence
<220>
   <223> Candidatus Mycoplasma haematoparvum clone 21533
<400> 145
   agcaaatggg attagatacc ccagtagtcc acgccgtaaa cgatgggtat taggtatttg        60
   atataagatt gagtgctgta gctaacgcgt taaatacccc gcctgggtag tatatatgca       120
   aatatgaaac tcaaagaaa                                                    139
<210> 146
   <211> 139
   <212> DNA
   <213> Artificial sequence
<220>
   <223> Candidatus Mycoplasma haematoparvum clone M63
<400> 146
   agcaaatggg attagatacc ccagtagtcc acgccgtaaa cgatgggtat taggtatttg        60
   atataagatt gagtgctgta gctaacgcgt taaatacccc gcctgggtag tatatatgca       120
   aatatgaaac tcaaagaaa                                                    139
<210> 147
   <211> 139
   <212> DNA
   <213> Artificial sequence
<220>
   <223> Candidatus Mycoplasma haematoparvum isolate 252.5a
<400> 147
   agcaaatggg attagatacc ccagtagtcc acgccgtaaa cgatgggtat taggtatttg        60
   atataagatt gagtgctgta gctaacgcgt taaatacccc gcctgggtag tatatatgca       120
   aatatgaaac tcaaagaaa                                                    139
<210> 148
   <211> 139
   <212> DNA
   <213> Artificial sequence
<220>
   <223> Candidatus Mycoplasma kahanei
<400> 148
   agcaaatggg attagatacc ccagtagtcc acgccgtaaa cgatgggtat taggtatttg 60
   gatgatgtct gagtgctgta gctaacgcgt taaatacccc gcctgggtag tatatatgca 120
   aatatgaaac tcaaagaaa 139
<210> 149
   <211> 139
   <212> DNA
   <213> Artificial sequence
<220>
   <223> Candidatus Mycoplasma kahanei isolate M46
<400> 149
   agcaaatggg attagatacc ccagtagtcc acgccgtaaa cgatgggtat taggtatttg 60
   gatgatgtct gagtgctgta gctaacgcgt taaatacccc gcctgggtag tatatatgca 120
   aatatgaaac tcaaagaaa 139
<210> 150
   <211> 143
   <212> DNA
   <213> Artificial sequence
<220>
   <223> Candidatus Mycoplasma haemozalophi isolate CSL 7801
<400> 150
   agcaaatggg attagatacc ccagtagtcc acgccgtaaa cgatgggtat taagtatttg 60
   attatcgtaa aattgagtgc tgtagctaac gcgttaaata ccccgcctgg gtagtatata 120
   tgcaaatatg aaactcaaag aaa 143
<210> 151
   <211> 139
   <212> DNA
   <213> Artificial sequence
<220>
   <223> Mycoplasma haemolama
<400> 151
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 152
   <211> 139
   <212> DNA
   <213> Artificial sequence
<220>
   <223> Candidatus Mycoplasma haemolamae clone GA1
<400> 152
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 153
   <211> 139
   <212> DNA
   <213> Artificial sequence
<220>
   <223> Candidatus Mycoplasma haemolamae clone Ohio-Tfles
<400> 153
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 154
   <211> 139
   <212> DNA
   <213> Artificial sequence
<220>
   <223> Candidatus Mycoplasma haemolamae clone OK-BR
<400> 154
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 155
   <211> 139
   <212> DNA
   <213> Artificial sequence
<220>
   <223> Candidatus Mycoplasma haemolamae clone OR1
<400> 155
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 156
   <211> 139
   <212> DNA
   <213> Artificial sequence
<220>
   <223> Candidatus Mycoplasma haemolamae partial
<400> 156
   agcaaatggg attagatacc ccagtagtcc acgccgtaaa cgatgggtat taggtatttg       60
   gtttaaaact gagtgctgta gctaacgcgt taaatacccc gcctgggtag tatatatgca      120
   aatatgaaac tcaaagaaa                                                   139
<210> 157
   <211> 139
   <212> DNA
   <213> Artificial sequence
<220>
   <223> Candidatus Mycoplasma haemolamae partial
<400> 157
   agcaaatggg attagatacc ccagtagtcc acgccgtaaa cgatgggtat taggtatttg       60
   gtttaaaact gagtgctgta gctaacgcgt taaatacccc gcctgggtag tatatatgca      120
   aatatgaaac tcaaagaaa                                                   139
<210> 158
   <211> 139
   <212> DNA
   <213> Artificial sequence
<220>
   <223> Candidatus Mycoplasma haemolamae partial
<400> 158
   agcaaatggg attagatacc ccagtagtcc acgccgtaaa cgatgggtat taggtatttg       60
   gtttaaaact gagtgctgta gctaacgcgt taaatacccc gcctgggtag tatatatgca      120
   aatatgaaac tcaaagaaa                                                   139
<210> 159
   <211> 139
   <212> DNA
   <213> Artificial sequence
<220>
   <223> Candidatus Mycoplasma haemolamae partial
<400> 159
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 160
   <211> 139
   <212> DNA
   <213> Artificial sequence
<220>
   <223> Candidatus Mycoplasma haemolamae partial
<400> 160
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 161
   <211> 139
   <212> DNA
   <213> Artificial sequence
<220>
   <223> Candidatus Mycoplasma haemolamae partial
<400> 161
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaagaaaa 139
<210> 162
   <211> 139
   <212> DNA
   <213> Artificial sequence
<220>
   <223> Candidatus Mycoplasma haemolamae partial
<400> 162
   agcaaatggg attagatacc ccagtagtcc acgccgtaaa cgatgggtac taggtatttg 60
   gtttaaaact gagtgctgta gctaacgcgt taaatacccc gcctgggtag tatatatgca 120
   aatatgaaac tcaaagaaa 139
<210> 163
   <211> 139
   <212> DNA
   <213> Artificial sequence
<220>
   <223> Candidatus Mycoplasma haemolamae partial
<400> 163
   agcaaatggg attagatacc ccagtagtcc acgccgtaaa cgatgggtat taggtatttg      60
   gtttaaaact gagtgctgta gctaacgcgt taaatacccc gcctgggtag tatatatgca     120
   aatatgaaac tcaaagaaa                                                  139
<210> 164
   <211> 139
   <212> DNA
   <213> Artificial sequence
<220>
   <223> Candidatus Mycoplasma haemolamae partial
<400> 164
   agcaaatggg attagatacc ccagtagtcc acgccgtaaa cgatgggtat taggtatttg      60
   gtttaaaact gagtgctgta gctaacgcgt taaatacccc gcctgggtag tatatatgca     120
   aatatgaaac tcaaagaaa                                                  139
<210> 165
   <211> 139
   <212> DNA
   <213> Artificial sequence
<220>
   <223> Candidatus Mycoplasma haemolamae strain Reading
<400> 165
   agcaaatggg attagatacc ccagtagtcc acgccgtaaa cgatgggtat taggtatttg      60
   gtttaaaact gagtgctgta gctaacgcgt taaatacccc gcctgggtag tatatatgca     120
   aatatgaaac tcaaagaaa                                                  139
<210> 166
   <211> 139
   <212> DNA
   <213> Artificial sequence
<220>
   <223> Candidatus Mycoplasma haemolamae strain SG-Ohio
<400> 166
   agcaaatggg attagatacc ccagtagtcc acgccgtaaa cgatgggtat taggtatttg      60
   gtttaaaact gagtgctgta gctaacgcgt taaatacccc gcctgggtag tatatatgca     120
   aatatgaaac tcaaagaaa                                                  139
<210> 167
   <211> 139
   <212> DNA
   <213> Artificial sequence
<220>
   <223> Candidatus Mycoplasma haemolamae strain VS-T194
<400> 167
   agcaaatggg attagatacc ccagtagtcc acgccgtaaa cgatgggtat taggtatttg 60
   gtttaaaact gagtgctgta gctaacgcgt taaatacccc gcctgggtag tatatatgca 120
   aatatgaaac tcaaagaaa 139
<210> 168
   <211> 139
   <212> DNA
   <213> Artificial sequence
<220>
   <223> Eperythrozoon suis
<400> 168
   agcaaatggg attagatacc ccagtagtcc acgccgtaaa cgatgggtat tagtcatttg 60
   aatttaagtt gagtgatgta gctaacgcgt taaatacccc gcctgggtag tatatatgca 120
   aatatgaaac tcaaagaaa 139
<210> 169
   <211> 140
   <212> DNA
   <213> Artificial sequence
<220>
   <223> Mycoplasma suis
<400> 169
   agcaaatggg attagatacc ccagtagtcc acgccgtaaa cgatgggtat tagtcatttg 60
   gatttaagac tgagtgatgt agctaacgcg ttaaataccc cgcctgggta gtatatatgc 120
   aaatatgaaa ctcaaagaaa 140
<210> 170
   <211> 139
   <212> DNA
   <213> Artificial sequence
<220>
   <223> Mycoplasma suis
<400> 170
   agcaaatggg attagatacc ccagtagtcc acgccgtaaa cgatgggtat tagtcatttg 60
   aatttaagtt aagtgatgta gctaacgcgt taaatacccc gcctgggtag tatatatgca 120
   aatatgaaac tcaaagaaa 139
<210> 171
   <211> 139
   <212> DNA
   <213> Artificial sequence
<220>
   <223> Mycoplasma suis KI3806
<400> 171
   agcaaatggg attagatacc ccagtagtcc acgccgtaaa cgatgggtat tagtcatttg 60
   aatttaagtt gagtgatgta gctaacgcgt taaatacccc gcctgggtag tatatatgca 120
   aatatgaaac tcaaagaaa 139
<210> 172
   <211> 139
   <212> DNA
   <213> Artificial sequence
<220>
   <223> Mycoplasma suis str. Illinois
<400> 172
   agcaaatggg attagatacc ccagtagtcc acgccgtaaa cgatgggtat tagtcatttg 60
   aatttaagtt gagtgatgta gctaacgcgt taaatacccc gcctgggtag tatatatgca 120
   aatatgaaac tcaaagaaa 139
<210> 173
   <211> 140
   <212> DNA
   <213> Artificial sequence
<220>
   <223> Mycoplasma suis strain Zhejiang
<400> 173
   agcaaatggg attagatacc ccagtagtcc acgccgtaaa cgatgggtat tagtcatttg 60
   gatttaagac tgagtgatgt agctaacgcg ttaaataccc cgcctgggta gtatatatgc 120
   aaatatgaaa ctcaaagaaa 140
<210> 174
   <211> 139
   <212> DNA
   <213> Artificial sequence
<220>
   <223> Eperythrozoon suis
<400> 174
   agcaaatggg attagatacc ccagtagtcc acgccgtaaa cgatgggtat tagtcatttg 60
   aatttaagtt gagtgatgta gctaacgcgt taaatacccc gcctgggtag tatatatgca 120
   aatatgaaac tcaaagaaa 139
<210> 175
   <211> 139
   <212> DNA
   <213> Artificial sequence
<220>
   <223> Eperythrozoon wenyonii
<400> 175
   agcaaatggg attagatacc ccagtagtcc acgccgtaaa cgatgggcat taggtatttg 60
   acatctagtc gagtgctgta gctaacgcgt taaatgcccc gcctgggtag tatatatgca 120
   aatatgaaac tcaaagaaa 139
<210> 176
   <211> 139
   <212> DNA
   <213> Artificial sequence
<220>
   <223> Eperythrozoon wenyonii
<400> 176
   agcaaatggg attagatacc ccagtagtcc acgccgtgaa cgatgggcat taggtatttg 60
   acatctagtc gagtgctgta gctaacgcgt taaatgcccc gcctgggtag tatatatgca 120
   aatatgaaac tcaaagaaa 139
<210> 177
   <211> 139
   <212> DNA
   <213> Artificial sequence
<220>
   <223> Mycoplasma wenyonii isolate cattle no. 43
<400> 177
   agcaaatggg attagatacc ccagtagtcc acgccgtaaa cgatgagcat tgggcatttg 60
   acatctggtc gagtgctgta gctaacgcgt taaatgctcc gcctgggtag tatatatgca 120
   aatatgaaac tcaaagaaa 139
<210> 178
   <211> 139
   <212> DNA
   <213> Artificial sequence
<220>
   <223> Mycoplasma wenyonii str. Massachusetts
<400> 178
   agcaaatggg attagatacc ccagtagtcc acgccgtaaa cgatgagcat tgggcatttg 60
   acatctggtc gagtgctgta gctaacgcgt taaatgctcc gcctgggtag tatatatgca 120
   aatatgaaac tcaaagaaa 139
<210> 179
   <211> 139
   <212> DNA
   <213> Artificial sequence
<220>
   <223> Mycoplasma wenyonii strain Langford
<400> 179
   agcacatggg atgagatacc ccagtagtcc acgccgtaaa cgatgagcat taggtatttg 60
   acatctggtc aagtgctgta gctaacgcgt taaatgcccc gcctgggtag tatatatgca 120
   aatatgaaac tcaaagaaa 139
<210> 180
   <211> 139
   <212> DNA
   <213> Artificial sequence
<220>
   <223> Candidatus Mycoplasma erythrocervae gene
<400> 180
   agcaaatggg attagatacc ccagtagtcc acgccgtaaa cgatgagcat taggtatttg 60
   gcattaggcc gagtgctgta gctaacgcgt taaatgctcc gcctgggtag tatatatgca 120
   aatatgaaac tcaaagaaa 139
<210> 181
   <211> 139
   <212> DNA
   <213> Artificial sequence
<220>
   <223> Candidatus Mycoplasma haemocervae gene
<400> 181
   atcaaatggg attagatacc ccagtagtcc acgccgtaaa cgatgagcat taggtatttg        60
   atgttatgtc gagtgctgta gctaacgcgt taaatgctcc gcctgggtag tatatatgca       120
   aatatgaaac tcaaagaaa                                                    139